(19) Europäisches Patentamt
European Patent Office
Office européen des brevets

(11) EP 2 634 180 A1

(12) EUROPEAN PATENT APPLICATION

(43) Date of publication:
**04.09.2013 Bulletin 2013/36**

(51) Int Cl.:
***C07D 307/33*** *(2006.01)* ***C07D 493/04*** *(2006.01)*
***C12P 17/04*** *(2006.01)*

(21) Application number: **12157752.2**

(22) Date of filing: **01.03.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Lonza Ltd**
**4052 Basel (CH)**

(72) Inventor: **The designation of the inventor has not yet been filed**

(74) Representative: **Winter, Brandl, Fürniss, Hübner, Röss, Kaiser, Polte - Partnerschaft**
**Alois-Steinecker-Strasse 22**
**85354 Freising (DE)**

(54) **Enzymatic process for the preparation of butyrolactones**

(57) The invention relates to a process for the preparation of a compound of formula

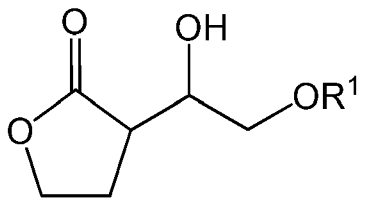

I,

wherein $R^1$ is selected from the group consisting of hydrogen, $C_{2-11}$-acyl, $C_{4-9}$-cycloalkanoyl, aryl, aralkyl, aroyl, hetaryl, hetaralkyl and hetaroyl, comprising a biotransformation of a compound of formula

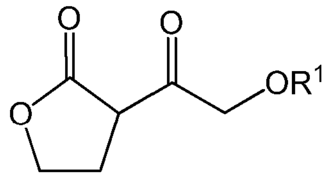

II,

wherein $R^1$ is as defined above, wherein said biotransformation is carried out using a polypeptide having aldo-keto reductase activity or a microorganism containing same.

EP 2 634 180 A1

SEQUENCE LISTING

<110> Lonza Ltd.

<120> Process for preparing lactones

<130> LO8204

<160> 13

<170> BiSSAP 1.0

<210> 1
<211> 14
<212> PRT
<213> Artificial Sequence

<220>
<221> SOURCE
<222> 1..14
<223> /mol_type="protein"
/note="Segment of aldo-keto reductase"
/organism="Artificial Sequence"

<220>
<221> VARIANT
<222> 2
<223> X is Lys, Pro, Ser or Gly

<220>
<221> VARIANT
<222> 3
<223> X is Thr, Gln, Ser, Arg, Leu or Lys

<220>
<221> VARIANT
<222> 4
<223> X is Ala, Cys, Gln, Ser or Thr

<220>
<221> VARIANT
<222> 6
<223> X is Ile, Leu, Val or Thr

<220>
<221> VARIANT
<222> 7
<223> X is Arg, His or Lys

<220>
<221> VARIANT
<222> 8
<223> X is Val, Ala, Ile or Thr

<220>
<221> VARIANT
<222> 10
<223> X is Pro, Cys, Val or Arg

<220>
<221> VARIANT
<222> 11
<223> X is Leu or Ile

<220>

```
<221> VARIANT
<222> 13
<223> X is Leu, Val, Phe or Ile

<400> 1
Ser Xaa Xaa Xaa Gly Xaa Xaa Xaa Ser Xaa Xaa Ile Xaa Gly
1               5                   10

<210> 2
<211> 11
<212> PRT
<213> Artificial Sequence

<220>
<221> SOURCE
<222> 1..11
<223> /mol_type="protein"
      /note="Segment of aldo-keto reductase"
      /organism="Artificial Sequence"

<220>
<221> VARIANT
<222> 2
<223> X is Gly, Val, Ile His or Ser

<220>
<221> VARIANT
<222> 3
<223> X is Asn, Thr or Arg

<220>
<221> VARIANT
<222> 4
<223> X is Cys, Phe, Glu, Leu or Ala

<220>
<221> VARIANT
<222> 5
<223> X is Ile Leu, Phe or Val

<220>
<221> VARIANT
<222> 7
<223> X is Thr or Val

<220>
<221> VARIANT
<222> 9
<223> X is Asn, Phe, Asp or Glu

<220>
<221> VARIANT
<222> 10
<223> X is Ser, Val, Phe, Ile or Ala

<400> 2
Gly Xaa Xaa Xaa Xaa Asp Xaa Ala Xaa Xaa Tyr
1               5                   10

<210> 3
<211> 16
<212> PRT
<213> Artificial Sequence

<220>
```

<221> SOURCE
<222> 1..16
<223> /mol_type="protein"
/note="Segment of aldo-keto reductase"
/organism="Artificial Sequence"

<220>
<221> VARIANT
<222> 2
<223> X is Arg, Gln, Ala, Lys, Glu or Thr

<220>
<221> VARIANT
<222> 3
<223> X is Lys, Arg or His

<220>
<221> VARIANT
<222> 4
<223> X is Leu or Met

<220>
<221> VARIANT
<222> 5
<223> X is deleted or Arg

<220>
<221> VARIANT
<222> 6
<223> X is Gln, Arg or Asp

<220>
<221> VARIANT
<222> 7
<223> X is Thr, Cys Met or Leu

<220>
<221> VARIANT
<222> 8
<223> X is Asp, Ser, Pro or Glu

<220>
<221> VARIANT
<222> 9
<223> X is Trp, Tyr, Lys or His

<220>
<221> VARIANT
<222> 10
<223> X is Ile, Val, Ala or Leu

<220>
<221> VARIANT
<222> 12
<223> X is Ile, Leu or Val

<220>
<221> VARIANT
<222> 13
<223> X is Leu, Phe, Tyr or Val
/

<220>
<221> VARIANT
<222> 14

<223> X is Tyr, Gln, Ile or Phe

<220>
<221> VARIANT
<222> 15
<223> X is Ile, Val, Cys, Met or Ala

<400> 3
Leu Xaa Xaa Xaa Xaa Xaa Xaa Xaa Xaa Xaa Asp Xaa Xaa Xaa Xaa His
1               5                   10                  15

<210> 4
<211> 14
<212> PRT
<213> Artificial Sequence

<220>
<221> SOURCE
<222> 1..14
<223> /mol_type="protein"
/note="Segment of aldo-keto reductase"
/organism="Artificial Sequence"

<220>
<221> VARIANT
<222> 2
<223> X is Lys, Pro, Ser or Gly

<220>
<221> VARIANT
<222> 3
<223> X is Thr, Gln, Ser, Arg, Leu or Lys

<220>
<221> VARIANT
<222> 4
<223> X is Ala, Cys, Gln, Ser or Thr

<220>
<221> VARIANT
<222> 6
<223> X is Ile, Leu, Val or Thr

<220>
<221> VARIANT
<222> 7
<223> X is Arg, His or Lys

<220>
<221> VARIANT
<222> 8
<223> X is Val, Ala, Ile or Thr

<220>
<221> VARIANT
<222> 10
<223> X is Pro, Cys, Val or Arg

<220>
<221> VARIANT
<222> 11
<223> X is Leu or Ile

<220>
<221> VARIANT

<222> 13
<223> X is Leu, Val, Phe or Ile

<400> 4
Leu Xaa Xaa Xaa Gly Xaa Xaa Xaa Pro Xaa Xaa Gly Xaa Gly
1               5                   10

<210> 5
<211> 47
<212> PRT
<213> Artificial Sequence

<220>
<221> SOURCE
<222> 1..47
<223> /mol_type="protein"
/note="segment of also-keto reductase"
/organism="Artificial Sequence"

<220>
<221> VARIANT
<222> 1
<223> X is an amino acid sequence of 10 to 30 amino acid residues

<220>
<221> VARIANT
<222> 3
<223> X is Lys, Pro, Ser or Gly

<220>
<221> VARIANT
<222> 4
<223> X is Thr, Gln, Ser, Arg, Leu or Lys

<220>
<221> VARIANT
<222> 5
<223> X is Ala, Cys, Gln, Ser or Thr

<220>
<221> VARIANT
<222> 7
<223> X is Ile, Leu, Val or Thr

<220>
<221> VARIANT
<222> 8
<223> X is Arg, His or Lys

<220>
<221> VARIANT
<222> 9
<223> X is Val, Ala, Ile or Thr

<220>
<221> VARIANT
<222> 11
<223> X is Pro, Cys, Val or Arg

<220>
<221> VARIANT
<222> 12
<223> X is Leu or Ile

<220>

<221> VARIANT
<222> 14
<223> X is Leu, Val, Phe or Ile

<220>
<221> VARIANT
<222> 16
<223> X is an amino acid sequence of 20 to 40 amino acid residues

<220>
<221> VARIANT
<222> 18
<223> X is Gly, Val, Ile His or Ser

<220>
<221> VARIANT
<222> 19
<223> X is Asn, Thr or Arg

<220>
<221> VARIANT
<222> 20
<223> X is Cys, Phe, Glu, Leu or Ala

<220>
<221> VARIANT
<222> 21
<223> X is Ile Leu, Phe or Val

<220>
<221> VARIANT
<222> 23
<223> X is Thr or Val

<220>
<221> VARIANT
<222> 25
<223> X is Asn, Phe, Asp or Glu

<220>
<221> VARIANT
<222> 26
<223> X is Ser, Val, Phe, Ile or Ala

<220>
<221> VARIANT
<222> 28
<223> X is an amino acid sequence of 10 to 40 amino acid residues

<220>
<221> VARIANT
<222> 30
<223> X is an amino acid sequence of 10 to 45 amino acid residues

<220>
<221> VARIANT
<222> 32
<223> X is Arg, Gln, Ala, Lys, Glu or Thr

<220>
<221> VARIANT
<222> 33
<223> X is Lys, Arg or His

<220>

<221> VARIANT
<222> 34
<223> X is Leu or Met

<220>
<221> VARIANT
<222> 35
<223> X is deleted or Arg

<220>
<221> VARIANT
<222> 36
<223> X is Gln, Arg or Asp

<220>
<221> VARIANT
<222> 37
<223> X is Thr, Cys Met or Leu

<220>
<221> VARIANT
<222> 38
<223> X is Asp, Ser, Pro or Glu

<220>
<221> VARIANT
<222> 39
<223> X is Trp, Tyr, Lys or His

<220>
<221> VARIANT
<222> 40
<223> X is Ile, Val, Ala or Leu

<220>
<221> VARIANT
<222> 42
<223> X is Ile, Leu or Val

<220>
<221> VARIANT
<222> 43
<223> X is Leu, Phe, Tyr or Val

<220>
<221> VARIANT
<222> 44
<223> X is Tyr, Gln, Ile or Phe

<220>
<221> VARIANT
<222> 45
<223> X is Ile, Val, Cys, Met or Ala

<220>
<221> VARIANT
<222> 47
<223> X is an amino acid sequence of 221 to 271 amino acid residues

<400> 5
```
Xaa Ser Xaa Xaa Xaa Gly Xaa Xaa Xaa Ser Xaa Xaa Ile Xaa Gly Xaa
1               5                   10                  15
Gly Xaa Xaa Xaa Xaa Asp Xaa Ala Xaa Xaa Tyr Xaa Lys Xaa Leu Xaa
            20                  25                  30
Xaa Xaa Xaa Xaa Xaa Xaa Xaa Xaa Asp Xaa Xaa Xaa Xaa His Xaa
```

35 40 45

<210> 6
<211> 47
<212> PRT
<213> Artificial Sequence

<220>
<221> SOURCE
<222> 1..47
<223> /mol_type="protein"
/note="segment of aldo-keto reductase"
/organism="Artificial Sequence"

<220>
<221> VARIANT
<222> 1
<223> X is an amino acid sequence of 10 to 30 amino acid residues

<220>
<221> VARIANT
<222> 3
<223> X is Lys, Pro, Ser or Gly

<220>
<221> VARIANT
<222> 4
<223> X is Thr, Gln, Ser, Arg, Leu or Lys

<220>
<221> VARIANT
<222> 5
<223> X is Ala, Cys, Gln, Ser or Thr

<220>
<221> VARIANT
<222> 7
<223> X is Ile, Leu, Val or Thr

<220>
<221> VARIANT
<222> 8
<223> X is Arg, His or Lys

<220>
<221> VARIANT
<222> 9
<223> X is Val, Ala, Ile or Thr

<220>
<221> VARIANT
<222> 11
<223> X is Pro, Cys, Val or Arg

<220>
<221> VARIANT
<222> 12
<223> X is Leu or Ile

<220>
<221> VARIANT
<222> 14
<223> X is Leu, Val, Phe or Ile

<220>

<221> VARIANT
<222> 16
<223> X is an amino acid sequence of 20 to 40 amino acid residues

<220>
<221> VARIANT
<222> 18
<223> X is Gly, Val, Ile His or Ser

<220>
<221> VARIANT
<222> 19
<223> X is Asn, Thr or Arg

<220>
<221> VARIANT
<222> 20
<223> X is Cys, Phe, Glu, Leu or Ala

<220>
<221> VARIANT
<222> 21
<223> X is Ile Leu, Phe or Val

<220>
<221> VARIANT
<222> 23
<223> X is Thr or Val

<220>
<221> VARIANT
<222> 25
<223> X is Asn, Phe, Asp or Glu

<220>
<221> VARIANT
<222> 26
<223> X is Ser, Val, Phe, Ile or Ala

<220>
<221> VARIANT
<222> 28
<223> X is an amino acid sequence of 10 to 40 amino acid residues

<220>
<221> VARIANT
<222> 30
<223> X is an amino acid sequence of 10 to 45 amino acid residues

<220>
<221> VARIANT
<222> 32
<223> X is Arg, Gln, Ala, Lys, Glu or Thr

<220>
<221> VARIANT
<222> 33
<223> X is Lys, Arg or His

<220>
<221> VARIANT
<222> 34
<223> X is Leu or Met

<220>

```
<221> VARIANT
<222> 35
<223> X is deleted or Arg

<220>
<221> VARIANT
<222> 36
<223> X is Gln, Arg or Asp

<220>
<221> VARIANT
<222> 37
<223> X is Thr, Cys Met or Leu

<220>
<221> VARIANT
<222> 38
<223> X is Asp, Ser, Pro or Glu

<220>
<221> VARIANT
<222> 39
<223> X is Trp, Tyr, Lys or His

<220>
<221> VARIANT
<222> 40
<223> X is Ile, Val, Ala or Leu

<220>
<221> VARIANT
<222> 42
<223> X is Ile, Leu or Val

<220>
<221> VARIANT
<222> 43
<223> X is Leu, Phe, Tyr or Val

<220>
<221> VARIANT
<222> 44
<223> X is Tyr, Gln, Ile or Phe

<220>
<221> VARIANT
<222> 45
<223> X is Ile, Val, Cys, Met or Ala

<220>
<221> VARIANT
<222> 47
<223> X is an amino acid sequence of 221 to 271 amino acid residues

<400> 6
Xaa Leu Xaa Xaa Xaa Gly Xaa Xaa Xaa Pro Xaa Xaa Gly Xaa Gly Xaa
1               5                   10                  15
Gly Xaa Xaa Xaa Xaa Asp Xaa Ala Xaa Xaa Tyr Xaa Lys Xaa Leu Xaa
            20                  25                  30
Xaa Xaa Xaa Xaa Xaa Xaa Xaa Xaa Asp Xaa Xaa Xaa Xaa His Xaa
        35                  40                  45

<210> 7
<211> 49
<212> PRT
```

```
<213> Artificial Sequence

<220>
<221> SOURCE
<222> 1..49
<223> /mol_type="protein"
      /note="segment of also-keto reductase"
      /organism="Artificial Sequence"

<220>
<221> VARIANT
<222> 1
<223> X is an amino acid sequence of 10 to 30 amino acid residues

<220>
<221> VARIANT
<222> 3
<223> X is Lys, Pro, Ser or Gly

<220>
<221> VARIANT
<222> 4
<223> X is Thr, Gln, Ser, Arg, Leu or Lys

<220>
<221> VARIANT
<222> 5
<223> X is Ala, Cys, Gln, Ser or Thr

<220>
<221> VARIANT
<222> 7
<223> X is Ile, Leu, Val or Thr

<220>
<221> VARIANT
<222> 8
<223> X is Arg, His or Lys

<220>
<221> VARIANT
<222> 9
<223> X is Val, Ala, Ile or Thr

<220>
<221> VARIANT
<222> 11
<223> X is Pro, Cys, Val or Arg

<220>
<221> VARIANT
<222> 12
<223> X is Leu or Ile

<220>
<221> VARIANT
<222> 14
<223> X is Leu, Val, Phe or Ile

<220>
<221> VARIANT
<222> 16
<223> X is an amino acid sequence of 20 to 40 amino acid residues

<220>
```

<221> VARIANT
<222> 18
<223> X is Gly, Val, Ile His or Ser

<220>
<221> VARIANT
<222> 19
<223> X is Asn, Thr or Arg

<220>
<221> VARIANT
<222> 20
<223> X is Cys, Phe, Glu, Leu or Ala

<220>
<221> VARIANT
<222> 21
<223> X is Ile Leu, Phe or Val

<220>
<221> VARIANT
<222> 23
<223> X is Thr or Val

<220>
<221> VARIANT
<222> 25
<223> X is Asn, Phe, Asp or Glu

<220>
<221> VARIANT
<222> 26
<223> X is Ser, Val, Phe, Ile or Ala

<220>
<221> VARIANT
<222> 28
<223> X is an amino acid sequence of 10 to 40 amino acid residues

<220>
<221> VARIANT
<222> 30
<223> X is an amino acid sequence of 10 to 45 amino acid residues

<220>
<221> VARIANT
<222> 32
<223> X is Arg, Gln, Ala, Lys, Glu or Thr

<220>
<221> VARIANT
<222> 33
<223> X is Lys, Arg or His

<220>
<221> VARIANT
<222> 34
<223> X is Leu or Met

<220>
<221> VARIANT
<222> 35
<223> X is deleted or Arg

<220>

<221> VARIANT
<222> 36
<223> X is Gln, Arg or Asp

<220>
<221> VARIANT
<222> 37
<223> X is Thr, Cys Met or Leu

<220>
<221> VARIANT
<222> 38
<223> X is Asp, Ser, Pro or Glu

<220>
<221> VARIANT
<222> 39
<223> X is Trp, Tyr, Lys or His

<220>
<221> VARIANT
<222> 40
<223> X is Ile, Val, Ala or Leu

<220>
<221> VARIANT
<222> 42
<223> X is Ile, Leu or Val

<220>
<221> VARIANT
<222> 44
<223> X is Tyr, Gln, Ile or Phe

<220>
<221> VARIANT
<222> 45
<223> X is Ile, Val, Cys, Met or Ala

<220>
<221> VARIANT
<222> 47
<223> X is an amino acid sequence of 70 to 100 amino acid residues

<220>
<221> VARIANT
<222> 49
<223> X is an amino acid sequence of 140 to 170 amino acid residues

<220>
<221> VARIANT
<222> 43
<223> X is Leu, Phe, Tyr or Val

<400> 7
```
Xaa Ser Xaa Xaa Xaa Gly Xaa Xaa Xaa Ser Xaa Xaa Ile Xaa Gly Xaa
1               5                   10                  15
Gly Xaa Xaa Xaa Xaa Asp Xaa Ala Xaa Xaa Tyr Xaa Lys Xaa Leu Xaa
            20                  25                  30
Xaa Xaa Xaa Xaa Xaa Xaa Xaa Xaa Asp Xaa Xaa Xaa Xaa His Xaa Val
        35                  40                  45
Xaa
```

<210> 8

<211> 49
<212> PRT
<213> Artificial Sequence

<220>
<221> SOURCE
<222> 1..49
<223> /mol_type="protein"
/note="segment of aldo-keto reductase"
/organism="Artificial Sequence"

<220>
<221> VARIANT
<222> 1
<223> X is an amino acid sequence of 10 to 30 amino acid residues

<220>
<221> VARIANT
<222> 3
<223> X is Lys, Pro, Ser or Gly

<220>
<221> VARIANT
<222> 4
<223> X is Thr, Gln, Ser, Arg, Leu or Lys

<220>
<221> VARIANT
<222> 5
<223> X is Ala, Cys, Gln, Ser or Thr

<220>
<221> VARIANT
<222> 7
<223> X is Ile, Leu, Val or Thr

<220>
<221> VARIANT
<222> 8
<223> X is Arg, His or Lys

<220>
<221> VARIANT
<222> 9
<223> X is Val, Ala, Ile or Thr

<220>
<221> VARIANT
<222> 11
<223> X is Pro, Cys, Val or Arg

<220>
<221> VARIANT
<222> 12
<223> X is Leu or Ile

<220>
<221> VARIANT
<222> 14
<223> X is Leu, Val, Phe or Ile

<220>
<221> VARIANT
<222> 16
<223> X is an amino acid sequence of 20 to 40 amino acid residues

<220>
<221> VARIANT
<222> 18
<223> X is Gly, Val, Ile His or Ser

<220>
<221> VARIANT
<222> 19
<223> X is Asn, Thr or Arg

<220>
<221> VARIANT
<222> 20
<223> X is Cys, Phe, Glu, Leu or Ala

<220>
<221> VARIANT
<222> 21
<223> X is Ile Leu, Phe or Val

<220>
<221> VARIANT
<222> 23
<223> X is Thr or Val

<220>
<221> VARIANT
<222> 25
<223> X is Asn, Phe, Asp or Glu

<220>
<221> VARIANT
<222> 26
<223> X is Ser, Val, Phe, Ile or Ala

<220>
<221> VARIANT
<222> 28
<223> X is an amino acid sequence of 10 to 40 amino acid residues

<220>
<221> VARIANT
<222> 30
<223> X is an amino acid sequence of 10 to 45 amino acid residues

<220>
<221> VARIANT
<222> 32
<223> X is Arg, Gln, Ala, Lys, Glu or Thr

<220>
<221> VARIANT
<222> 33
<223> X is Lys, Arg or His

<220>
<221> VARIANT
<222> 34
<223> X is Leu or Met

<220>
<221> VARIANT
<222> 35
<223> X is deleted or Arg

<220>
<221> VARIANT
<222> 36
<223> X is Gln, Arg or Asp

<220>
<221> VARIANT
<222> 37
<223> X is Thr, Cys Met or Leu

<220>
<221> VARIANT
<222> 38
<223> X is Asp, Ser, Pro or Glu

<220>
<221> VARIANT
<222> 39
<223> X is Trp, Tyr, Lys or His

<220>
<221> VARIANT
<222> 40
<223> X is Ile, Val, Ala or Leu

<220>
<221> VARIANT
<222> 42
<223> X is Ile, Leu or Val

<220>
<221> VARIANT
<222> 44
<223> X is Tyr, Gln, Ile or Phe

<220>
<221> VARIANT
<222> 45
<223> X is Ile, Val, Cys, Met or Ala

<220>
<221> VARIANT
<222> 47
<223> X is an amino acid sequence of 70 to 100 amino acid residues

<220>
<221> VARIANT
<222> 49
<223> X is an amino acid sequence of 140 to 170 amino acid residues

<220>
<221> VARIANT
<222> 43
<223> X is Leu, Phe, Tyr or Val

<400> 8
```
Xaa Leu Xaa Xaa Xaa Gly Xaa Xaa Xaa Pro Xaa Xaa Gly Xaa Gly Xaa
1               5                   10                  15
Gly Xaa Xaa Xaa Xaa Asp Xaa Ala Xaa Xaa Tyr Xaa Lys Xaa Leu Xaa
            20                  25                  30
Xaa Xaa Xaa Xaa Xaa Xaa Xaa Xaa Asp Xaa Xaa Xaa Xaa His Xaa Val
        35                  40                  45
Xaa
```

<210> 9
<211> 26
<212> PRT
<213> Artificial Sequence

<220>
<221> SOURCE
<222> 1..26
<223> /mol_type="protein"
/note="segment of aldo-keto reductase"
/organism="Artificial Sequence"

<400> 9
Gln Asn Glu Glu Ser Glu Ile Trp Ile Gly Glu Trp Met Ala Ser Arg
1               5                   10                  15
Lys Leu Arg Asp Gln Ile Val Ile Ala Thr
            20                  25

<210> 10
<211> 178
<212> PRT
<213> Artificial Sequence

<220>
<221> SOURCE
<222> 1..178
<223> /mol_type="protein"
/note="segment of aryl-alcohol dehydrogenase YNL331C"
/organism="Artificial Sequence"

<400> 10
Thr Asp Leu Phe Lys Pro Leu Pro Glu Pro Pro Thr Glu Leu Gly Arg
1               5                   10                  15
Leu Arg Val Leu Ser Lys Thr Ala Gly Ile Arg Val Ser Pro Leu Ile
            20                  25                  30
Leu Gly Gly Ala Ser Ile Gly Asp Ala Trp Ser Gly Phe Met Gly Ser
        35                  40                  45
Met Asn Lys Glu Gln Ala Phe Glu Leu Leu Asp Ala Phe Tyr Glu Ala
    50                  55                  60
Gly Gly Asn Cys Ile Asp Thr Ala Asn Ser Tyr Gln Asn Glu Glu Ser
65                  70                  75                  80
Glu Ile Trp Ile Gly Glu Trp Met Ala Ser Arg Lys Leu Arg Asp Gln
                85                  90                  95
Ile Val Ile Ala Thr Lys Phe Thr Gly Asp Tyr Lys Lys Tyr Glu Val
            100                 105                 110
Gly Gly Gly Lys Ser Ala Asn Tyr Cys Gly Asn His Lys Arg Ser Leu
        115                 120                 125
His Val Ser Val Arg Asp Ser Leu Arg Lys Leu Gln Thr Asp Trp Ile
    130                 135                 140
Asp Ile Leu Tyr Ile His Trp Trp Asp Tyr Met Ser Ser Ile Glu Glu
145                 150                 155                 160
Val Met Asp Ser Leu His Ile Leu Val Gln Gln Gly Lys Val Leu Tyr
                165                 170                 175
Leu Gly

<210> 11
<211> 382
<212> PRT
<213> Saccharomyces cerevisiae

<220>
<221> SOURCE
<222> 1..382

```
<223> /mol_type="protein"
      /note="aryl-alcohol dehydrogenase YNL331C"
      /organism="Saccharomyces cerevisiae"

<400> 11
Thr Asp Leu Phe Lys Pro Leu Pro Glu Pro Pro Thr Glu Leu Gly Arg
1               5                   10                  15
Leu Arg Val Leu Ser Lys Thr Ala Gly Ile Arg Val Ser Pro Leu Ile
            20                  25                  30
Leu Gly Gly Ala Ser Ile Gly Asp Ala Trp Ser Gly Phe Met Gly Ser
        35                  40                  45
Met Asn Lys Glu Gln Ala Phe Glu Leu Leu Asp Ala Phe Tyr Glu Ala
    50                  55                  60
Gly Gly Asn Cys Ile Asp Thr Ala Asn Ser Tyr Gln Asn Glu Glu Ser
65                  70                  75                  80
Glu Ile Trp Ile Gly Glu Trp Met Ala Ser Arg Lys Leu Arg Asp Gln
                85                  90                  95
Ile Val Ile Ala Thr Lys Phe Thr Gly Asp Tyr Lys Lys Tyr Glu Val
            100                 105                 110
Gly Gly Gly Lys Ser Ala Asn Tyr Cys Gly Asn His Lys Arg Ser Leu
        115                 120                 125
His Val Ser Val Arg Asp Ser Leu Arg Lys Leu Gln Thr Asp Trp Ile
    130                 135                 140
Asp Ile Leu Tyr Ile His Trp Trp Asp Tyr Met Ser Ser Ile Glu Glu
145                 150                 155                 160
Val Met Asp Ser Leu His Ile Leu Val Gln Gln Gly Lys Val Leu Tyr
                165                 170                 175
Leu Gly Val Ser Asp Thr Pro Ala Trp Val Val Ser Ala Ala Asn Tyr
            180                 185                 190
Tyr Ala Thr Ser His Gly Lys Thr Pro Phe Ser Val Tyr Gln Gly Lys
        195                 200                 205
Trp Asn Val Leu Asn Arg Asp Phe Glu Arg Asp Ile Ile Pro Met Ala
    210                 215                 220
Arg His Phe Gly Met Ala Leu Ala Pro Trp Asp Val Met Gly Gly Gly
225                 230                 235                 240
Arg Phe Gln Ser Lys Lys Ala Met Glu Glu Arg Lys Lys Asn Gly Glu
                245                 250                 255
Gly Leu Arg Thr Phe Val Gly Gly Pro Glu Gln Thr Glu Leu Glu Val
            260                 265                 270
Lys Ile Ser Glu Ala Leu Thr Lys Ile Ala Glu Glu His Gly Thr Glu
        275                 280                 285
Ser Val Thr Ala Ile Ala Ile Ala Tyr Val Arg Ser Lys Ala Lys Asn
    290                 295                 300
Val Phe Pro Leu Ile Gly Gly Arg Lys Ile Glu His Leu Lys Gln Asn
305                 310                 315                 320
Ile Glu Ala Leu Ser Ile Lys Leu Thr Pro Glu Gln Ile Glu Tyr Leu
                325                 330                 335
Glu Ser Ile Val Pro Phe Asp Val Gly Phe Pro Lys Ser Leu Ile Gly
            340                 345                 350
Asp Asp Pro Ala Val Thr Lys Lys Leu Ser Pro Leu Thr Ser Met Ser
        355                 360                 365
Ala Arg Ile Ala Phe Asp Asn Lys Leu Ala Ala Ala Leu Glu
    370                 375                 380

<210> 12
<211> 1131
<212> DNA
<213> Saccharomyces cerevisiae

<220>
<221> source
<222> 1..1131
<223> /mol_type="DNA"
      /note="CDS for aryl-alcohol dehydrogenase YNL331C"
      /organism="Saccharomyces cerevisiae"
```

<220>
<221> gene
<222> 1..1131
<223> /product="aryl-alcohol dehydrogenase YNL331C"

<400> 12
atgactgact tgtttaaacc tctacctgaa ccacctaccg aattgggacg tctcagggtt 60

ctttctaaaa ctgccggcat aagggtttca ccgctaattc tgggaggagc ttcaatcggc 120

gacgcatggt caggctttat gggctctatg aataaggaac aggcctttga acttcttgat 180

gctttttatg aagctggagg taattgtatt gatactgcaa acagttacca aaatgaagag 240

tcagagattt ggataggtga atggatggca tcaagaaaac tgcgtgacca gattgtaatt 300

gccaccaagt ttaccggaga ttataagaag tatgaagtag gtggtggtaa aagtgccaac 360

tactgtggta atcacaagcg tagtttacat gtgagtgtga gggattctct ccgcaaattg 420

caaactgatt ggattgatat actttacatt cactggtggg attatatgag ttcaatcgaa 480

gaagttatgg atagtttgca tattttagtt cagcagggca aggtcctata tttaggagta 540

tctgatacac ctgcttgggt tgtttctgcg gcaaattact acgctacatc tcatggtaaa 600

actcctttta gcgtctatca aggtaaatgg aatgtattga acagggactt tgagcgtgat 660

attattccaa tggctaggca ttttggtatg gctctagccc catgggatgt catgggaggt 720

ggaagatttc agagtaaaaa agcaatggaa gaacggaaga agaatggaga gggtctgcgt 780

acttttgtgg gtggccccga acaaacagaa ttggaggtta aaatcagcga agcattgact 840

aaaattgctg aggaacatgg aacagagtct gttactgcta tcgctattgc ctatgttcgc 900

tctaaagcga aaaatgtttt cccattgatt ggaggaagga aaattgaaca tctcaagcag 960

aacattgagg ctttgagtat taaattaaca ccggaacaaa tagaatacct ggaaagtatt 1020

gttccttttg atgttggctt tcccaaaagt ttaataggag atgacccagc ggtaaccaag 1080

aagctttcac ccctcacatc gatgtctgcc aggatagctt ttgacaatta g 1131

<210> 13
<211> 1170
<212> DNA
<213> Saccharomyces cerevisiae

<220>
<221> source
<222> 1..1170
<223> /mol_type="DNA"
/note="CDS for aryl-alcohol dehydrogenase YNL331C"
/organism="Saccharomyces cerevisiae"

<220>
<221> gene
<222> 1..1170
<223> /product="aryl-alcohol dehydrogenase YNL331C having a His-Tag "

<400> 13
atgactgact tgtttaaacc tctacctgaa ccacctaccg aattgggacg tctcagggtt 60

```
ctttctaaaa ctgccggcat aagggtttca ccgctaattc tgggaggagc ttcaatcggc     120
gacgcatggt caggctttat gggctctatg aataaggaac aggcctttga acttcttgat     180
gctttttatg aagctggagg taattgtatt gatactgcaa acagttacca aaatgaagag     240
tcagagattt ggataggtga atggatggca tcaagaaaac tgcgtgacca gattgtaatt     300
gccaccaagt ttaccggaga ttataagaag tatgaagtag gtggtggtaa aagtgccaac     360
tactgtggta atcacaagcg tagtttacat gtgagtgtga gggattctct ccgcaaattg     420
caaactgatt ggattgatat actttacatt cactggtggg attatatgag ttcaatcgaa     480
gaagttatgg atagtttgca tattttagtt cagcagggca aggtcctata tttaggagta     540
tctgatacac ctgcttgggt tgtttctgcg gcaaattact acgctacatc tcatggtaaa     600
actcctttta gcgtctatca aggtaaatgg aatgtattga acagggactt tgagcgtgat     660
attattccaa tggctaggca ttttggtatg gctctagccc catgggatgt catgggaggt     720
ggaagatttc agagtaaaaa agcaatggaa gaacggaaga agaatggaga gggtctgcgt     780
acttttgtgg gtggccccga acaaacagaa ttggaggtta aaatcagcga agcattgact     840
aaaattgctg aggaacatgg aacagagtct gttactgcta tcgctattgc ctatgttcgc     900
tctaaagcga aaaatgtttt cccattgatt ggaggaagga aaattgaaca tctcaagcag     960
aacattgagg ctttgagtat taaattaaca ccggaacaaa tagaatacct ggaaagtatt    1020
gttccttttg atgttggctt tcccaaaagt ttaataggag atgacccagc ggtaaccaag    1080
aagctttcac ccctcacatc gatgtctgcc aggatagctt ttgacaataa gcttgcggcc    1140
gcactcgagc accaccacca ccaccactga                                     1170
```

**Description**

The present invention relates to a process for the preparation of a compound of formula

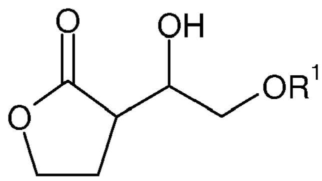

I,

wherein $R^1$ is selected from the group consisting of hydrogen, $C_{2-11}$-acyl, $C_{4-9}$-cycloalcanoyl, aryl, aralkyl, aroyl, hetaryl, hetaralkyl and hetaroyl.

The present invention also relates to a process for the preparation of a compound of formula

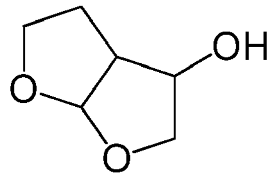

III,

more preferably of formula

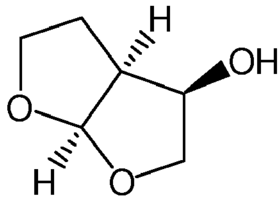

IIIa.

which may be obtained from the compound of formula I and which is an important intermediate in the production of pharmaceuticals such as the compound of the formula

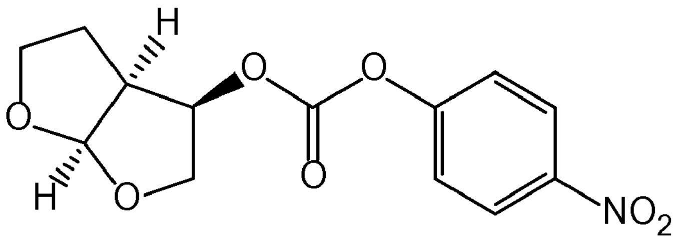

VII

or the compound of formula

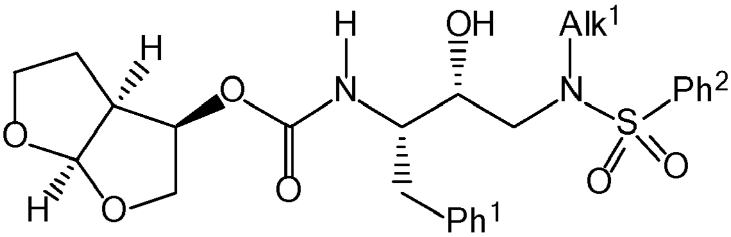

VIII,

derived therefrom, wherein $Alk^1$ is a linear or branched $C_{1-8}$-alkyl group, $Ph^1$ is phenyl and $Ph^2$ is 4-amino-phenyl, or

wherein $Alk^1$ is a linear or branched $C_{1-8}$-alkyl group, $Ph^1$ is 4-[(2-methyl-1,3-thiazol-4-yl)methoxy]phenyl and $Ph^2$ is 1,3-benzodioxol-5-yl, or any physiologically acceptable salt thereof.

Active pharmaceutical ingredients (API) of formula VIII

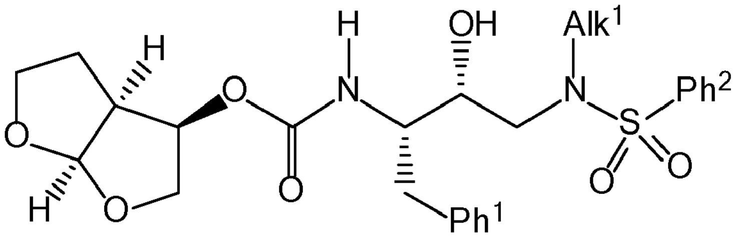

VIII,

(Darunavir or TMC-114, wherein $Alk^1$ is iso-butyl, $Ph^1$ is phenyl and $Ph^2$ is 4-amino-phenyl; Brecanavir or GW640385, wherein $Alk^1$ is iso-butyl, $Ph^1$ is 4-[(2-methyl-1,3-thiazol-4-yl)methoxy]phenyl and $Ph^2$ is 1,3-benzodioxol-5-yl) are powerful anti-HIV drugs prescribed for patients with drug-resistant AIDS and are part of the highly active antiretroviral therapy (HAART) recommended by the American National Institutes of Health (NIH) and World Health Organization (WHO). One of the key intermediates for the preparation of either Darunavir and Brecanavir is the hexahydro-furo[2,3-b]furan-3-ol (6H-furofuranol) moiety of formula IIIa,

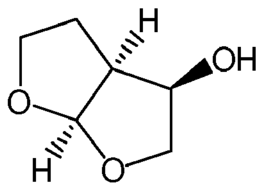

IIIa

which can be introduced for example via the compound of formula

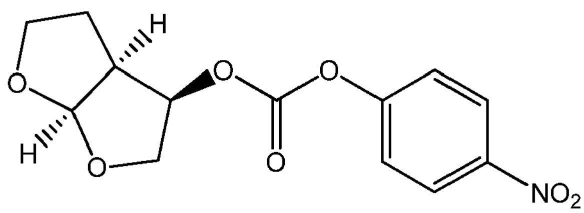

VII

The downstream processes and examples starting with the compound of formula III to obtain pharmaceutically active compounds are disclosed in WO/2005/063209, WO/2007/126812 and US2004/0127727, which all are incorporated herein by reference regarding said downstream processes to obtain the API and respective formulations for administration to patients.

EP-A 1532127 and EP-A 1589018 disclose different strategies to obtain a compound of formula III.

Among other methods, EP-A 1589018 discloses a process for the preparation of a lactone of formula

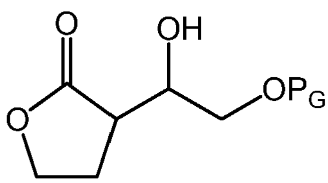

wherein $P_G$ is a hydroxyl protecting group, which is used to obtain a compound of formula

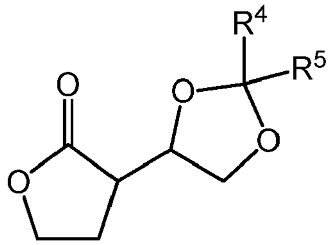

wherein $R_4$ and $R_5$ are the same or different and each is a hydrogen atom, a lower alkyl group, a lower alkoxy group or a phenyl group. This compound is subjected to further reduction to obtain a compound of formula

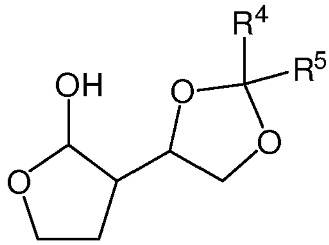

which after deprotection and cyclization gives the compound of formula III

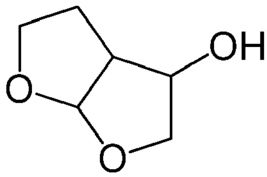

III

In EP-A 1589018 optical resolution of the racemic mixture of the compound of formula III is performed to obtain the compound of the formula

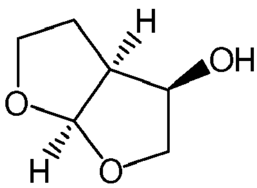

IIIa

The object of the present invention was to provide a simple, low-cost and more economical process for preparing the compounds of formula I and III to provide optically pure intermediates without optical resolution, for example for the preparation of pharmaceutically active ingredients such as Darunavir.

(1)

This object has been achieved by a process for the preparation of a compound of formula

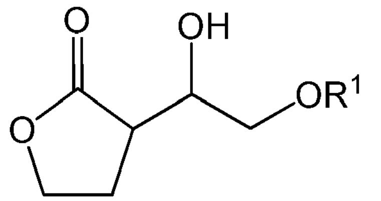

I,

wherein $R^1$ is selected from the group consisting of hydrogen, $C_{2-11}$-acyl, $C_{4-9}$-cycloalkanoyl, aryl, aralkyl, aroyl, hetaryl, hetaralkyl and hetaroyl, comprising a biotransformation of a compound of formula

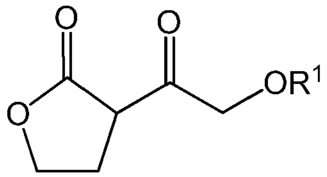

II,

wherein $R^1$ is as defined above, wherein said biotransformation is carried out using a polypeptide having aldo-keto reductase activity or a microorganism containing same.

In the residue definitions, generally $C_{n-m}$ indicates a group comprising n to m carbon atoms. For example $C_{1-4}$ and $C_{2-11}$ refer to a group having 1 to 4, or 2 to 11 carbon atoms, respectively.

$C_{1-10}$-alkyl refers to a linear or branched alkyl group having 1 to 8 carbon atoms, optionally substituted with one or more halogen atoms. Halogen may be F, Cl, Br and I. Examples of $C_{1-10}$-alkyl are methyl, ethyl, propyl, isopropyl, butyl, i.e. n-butyl, sec-butyl, iso-butyl or tert-butyl; pentyl, hexyl, heptyl, octyl, nonyl, decyl, bromomethyl, chloromethyl, fluoromethyl, dibromomethyl, dichloromethyl, difluoromethyl, up to perfluoroalykl, such as perfluoromethyl or perfluoroethyl. Preferably $C_{1-10}$-alkyl is selected from $C_{1-4}$-alkyl, i.e. methyl, ethyl, propyl, isopropyl, butyl, i.e. *n*-butyl, sec-butyl, *iso*-bytyl, *tert*-butyl; bromomethyl, chloromethyl, fluoromethyl, dibromomethyl, dichloromethyl, difluoromethyl, perfluoromethyl or perfluoroethyl.

$C_{1-4}$-alkoxy refers to a $C_{1-4}$-alkyl group as defined above, which is attached via an oxygen atom. Preferably $C_{1-4}$-alkoxy is selected from methoxy, ethoxy, propoxy, isopropoxy, butoxy, i.e. *n*-butoxy, sec-butoxy or *tert*-butoxy.

$C_{2-11}$-acyl refers to a linear or branched $C_{1-10}$-alkyl group as defined above, which is attached via a carbonyl group, for example acetyl, propionyl, butyryl, pentylcarbonyl, hexylcarbonyl, heptylcarbonyl, octylcarbonyl, nonanylcarbonyl, decylcarbonyl, 2-bromoacetyl, 2-chloroacetyl, 2-fluoroacetyl, dibromoacetyl, dichloroacetyl, difluoroacetyl, tribromoacetyl, trichloroacetyl, trifluoroacetyl, 2-bromopropionyl, 3-bromopropionyl, 2-chloropropionyl, 3-chloropropionyl, 2-fluoropropionyl, 3-fluoropropionyl, 2,2-dibromopropionyl, 2,3-dibromopropionyl, 2,2-dichloropropionyl, 2,3-dichloropropionyl, 2,2-difluoropropionyl, 2,3-difluoropropionyl, perbromopropionyl, perchloropropionyl or perfluoropropionyl.

$C_{4-9}$-cycloalkanoyl refers to a $C_{3-8}$-cycloalkyl group, for example cyclopropyl, cyclobutyl, cyclopentyl, cyclohexyl, cycloheptyl or cyclooctyl, which is attached via a carbonyl group. The cycloylkyl group may be unsubstituted or substituted by one or more halogen atoms. Halogen may be F, Cl, Br and I.

Aryl means phenyl or naphthyl, optionally substituted with one to three substituents selected from halogen atoms, $C_{1-4}$-alkyl as defined above, $C_{1-4}$-alkoxy as defined above, cyano and nitro groups. Halogen may be F, Cl, Br or I. Examples of aryl are phenyl, 4-halophenyl, 3,4-dihalophenyl, 2,5-dihalophenyl, 4-$C_{1-4}$-alkyl-phenyl, 3,4-di-$C_{1-4}$-alkyl-phenyl or 3,5-di-$C_{1-4}$-alkyl-phenyl.

Aralkyl refers to aryl as defined above, which is attached via a methylene or ethylene group. Examples for aralkyl are benzyl, 2-phenethyl, 2-methylbenzyl, 3-methylbenzyl, 4-methylbenzyl, 2,4-dimethylbenzyl, 2-(4-ethylphenyl) ethyl, 1-naphthalenemethyl or 2-naphthalenemethyl.

Aroyl refers to aryl as defined above, which is attached via a carbonyl group. Aroyl is for example benzoyl, halobenzoyl such as 4-bromobenzoyl or 4-chlorobenzyloxy, naphthalene-1-carbonyl, or halonaphthalene-1-carbonyl.

Hetaryl means a 5- or 7-membered heterocyclic aromatic group having independently one to three heteroatoms selected from oxygen, nitrogen and sulfur in the ring. The ring may be either unsubstituted or substituted by one or more halogen atoms or one or more $C_{1-4}$-alkyl groups as defined above. Halogen may be F, Cl, Br and I. Examples of hetaryl are 2-furanyl, 3-furanyl, 2-thienyl, 3-thienyl, 2-benzo[b]furanyl, 2-benzo[b]thienyl, 2-pyridinyl, 3-pyridinyl or 4-pyridinyl.

Hetaralkyl refers to a hetaryl group as defined above, which is attached via a methylene or ethylene group. Examples for hetarylalkyl are furylmethyl, thienylmethyl, pyrazolylmethyl, imidazolylmethyl, 1,2,3- and 1,2,4-triazolylmethyl, isoxazolylmethyl, thiazolylmethyl, isothiazolylmethyl, 1,2,3-, 1,3,4-, 1,2,4- and 1,2,5-oxadiazolylmethyl, azepinylmethyl, pyrrolylmethyl, pyridylmethyl, pyridazinylmethyl, pyrimidinyl-methyl, pyrazinylmethyl, 1,3,5-, 1,2,4- and 1,2,3-triazinylmethyl, 1,2,4-, 1,3,2-, 1,3,6- and 1,2,6-oxazinylmethyl, oxepinylmethyl, thiepinylmethyl and 1,2,4-diazepinylmethyl.

Hetaroyl means hetaryl as defined above, which is attached via a carbonyl group. Suitable hetaroyl groups are for example 2- or 3-thenoyl, nicotinoyl, 2- or 3-furoyl.

Chiral diastereoisomers obtainable from a biotransformation as outlined above are:

the anti diastereoisomers (*S,R* and *R,S*) of formulae Ia and Ib:

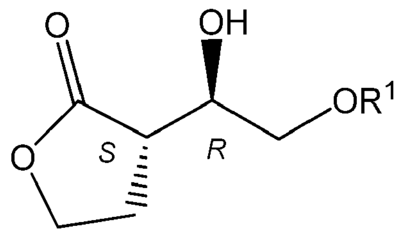

Ia,

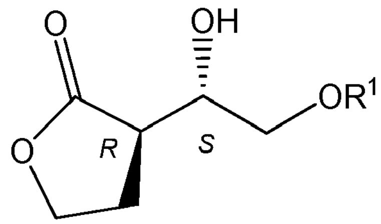

Ib,

and the syn-diastereoisomers (*R,R* and *S,S*) of formulae Ic and Id:

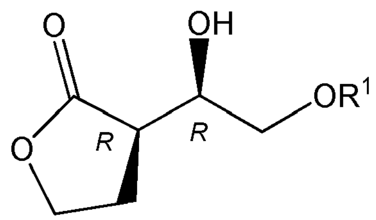

Ic

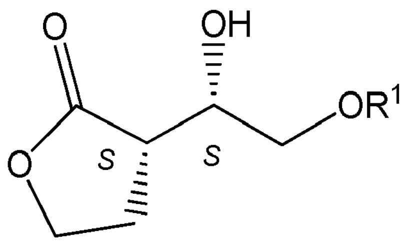

Id,

respectively, wherein $R^1$ is as defined above.

(2)

Currently, the compound of formula Ia is the most preferred diastereoisomer. The present invention, therefore, also provides the process as defined above for the specific preparation of a compound of formula

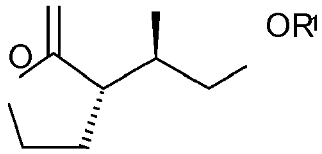

Ia,

wherein $R^1$ is as defined above. Preferably, $R^1$ is selected from hydrogen, $C_{2-11}$-acyl, aryl, aralkyl, aroyl, hetaryl, hetaralkyl and hetaroyl.

The term polypeptide having aldo-keto reductase activity (hereinafter also referred to as aldo-keto reductase) refers to a polypeptide having the activity of an enzyme of the aldo-keto reductase superfamily. These polypeptides have been found to be capable of reducing ketolactones of the formula II to the corresponding hydroxylactones of formula I. Members of the aldo-keto reductase superfamily typically use cofactors NADH or NADPH as the hydride source. Typically, aldo-keto reductases useful in the process of the invention are polypeptides or enzymes comprising 271 to 426 amino acid residues.

The enzymatic reduction of the compound of formula II to obtain a compound of formula I can be performed, for example, by use of a purified polypeptide having said aldo-keto reductase activity, a microorganism expressing a polypeptide having said aldo-keto reductase activity, preferably a microorganism which is able to utilize compounds of formula II as sole carbon source, or a cell free extract of a microorganism expressing said polypeptide having aldo-keto reductase activity. In a preferred embodiment of the invention, the polypeptide is used in isolated form, either free or immobilized on a carrier, as a cell free enzyme extract, for example prepared from crude lysate, as crude lysate from whole cell suspensions, or within a whole cell suspension.

Polypeptides having aldo-keto reductase activity useful in the invention may be derived from prokaryotic as well as eukaryotic cells such as fungal cells, in particular yeasts. Examples thereof are disclosed, for example, in Jez, J.M. and Penning, T.M., Chem. Biol. Interact. 130-132, 2001, 499-525, and Jez, J.M.; Flynn, T.G. and Penning, T.M., Biochem. Pharmacol. 54, 1997, 639-647. The term "aldo-keto reductase or "polypeptide having aldo-keto reductase activity" is intended to include variants or biologically active fragments of naturally occuring enzymes as long as they have said aldo-keto reductase activity. The polypeptides may be used in glycosylated or non-glycosylated form and may be produced recombinantly or non-recombinantly.

Polypeptides having aldo-keto reductase activity capable of reducing a compound of formula II, wherein R is as defined above, to a compound of formula I can be obtained from bacteria of the genera selected from the group consisting of abitrophia, achromobacter, acidovorax, acinetobacter, aerococcus, aeromonas, agrobacterium, alcaligenes, alishwanella, alloicoccis, ammoniphilus, amphibacillus, amycolatopsis, aneurinbacillus, arcanobacterium, arthrobacter, bacillus, balneatrix, bergeyella, brenneria, brevibacillus, brevibacterium, brevundimonas, budvicia, burkholderia, buttiauxella, cedecea, cellulomonas, cellulosimicrobium, chromobacterium, chrysobacterium, citrobacter, comamonas, corynebacterium, curtobacterium, delftia, dermabacter, dermacoccus, dolosicoccus, edwardsiella, empedobacter, enterobacter, enterococcus, erwinia, erysipelothrix, escherichia, ewingella, exiguobacterium, facklamia, flavimonas, flavobacterium, gemella, gallobicatella, granulicatella, hafnia, halomonas, helcoccus, klebsiella, kluyvera, kocuria, kytococcus, lactococcus, leclercia, legionella, leifsonia, leminorella, leuconostoc, listeria, macrococcus, methylobacterium, microbacterium, micrococcus, micropolyspora, moellerera, moraxella, morganella, mycobacterium, myoides, nresterenkonia, nocardia, nocardiopsis, obesumbacterium, ochromobacterium, oerskovia, olligella, paenibacillus, poandoraea, pantoea, pasteurella, pectobacterium, pediococcus, pedobacter, photohabdus, plesiomonas, pragia, proteus, providencia, pseudomonas, psychobacter, rahnella, ralstonia, raoutella, rhizobium, rhodococcus, roseomonas, rothia, saccharomonospora, saccharopolyspora, salmonella, serratia, shewanella, sphingobacterium, sphingomonas, sodalis, staphylococcus, stenotrophomonas, streptococcus, streptomyces, tatumella, thermoactinomyces, thermobacillus, trabulsiella, vagococcus, virgibacillus, weeksella, xenorhabdus, yersinia, and yonekella.

Preferred yeasts and fungi from which polypeptides having aldo-keto reductase activity can be obtained from genera selected from the group consisting of absidia, acremonium, alternaria, amblysporium, aphanocladium, arthrinium, arthrobotrys, arthroderma, arthrographis, ascotricha, aspergillus, asteromyces, aureobasidium, basipetospora, beauveria, bipolaris, bispora, botryosporium, botrytis, byssochlamys, caldariomyces, calcarisporium, cephaliophora, ceratocystis, cerebella, cercospora, chaetmium, chromelosporium, chrysinilia, chrysosporium, cladiophialophora, cladosporium, collectrichum, cunninghamella, curvularia, cylindrocarpon, cylindrocladium, dicyma, dreschlera, echinobotryum, emericella, emmonsia, epicoccum, epidermophyton, eupenicillium, eurotium, exophiala, exserihilum, fusarium, geomyces, geotrichum, gliocladium (also referred as clonobotrys), gliomastix, gonatobotryum, graphium, helminthosporium, hormographiella, humicola, hyalodendron, lecythophora, leptographium, leptosphaeria, leptosphaerulina, malbranchea, memnoniella, metarhizium, microspaerospsis, microsporium, monodictys, mortierella, mucor, mycothypha, myrothecium, nectria, neosartorya, nigrospora, oedocephalum, oidiodendron, ophiostoma, paecilomyces, papulaspora, penicillium, pericornia, pestaliopsis, phialocephala, philophora, phoma, phragmopcephala, pithomyces, polypaecilum, polythricium, rhinocladiella, rhizomucor, rhizopus, rhodotorula, scolecobasidium (also referred as ochroconis), scopulariopsis, scytalidium, sepedonium, sordaria, spegazzinia, sporobolomyces, sporothrix, sporotrichum, stachybotrys, staphylotrichum, stemphylium, syncephalastrum, taeniolella, talaromyces, tetraploa, thamnidium, thysanospora, tilletiopsis, torula, torulomyces, trichiconiella, trichoderma, tricophyton, tricothecium, tritirachium, ulocladium, verticillium, verticicladiella, wallemia, and xeromyces.

Microorganisms expressing polypeptides having aldo-keto reductase activity suitable for use in the process of the present invention can be obtained by contacting a candidate microorganism with a compound of formula II, wherein $R^1$ is selected from the group consisting of hydrogen, $C_{2-11}$-acyl, $C_{4-9}$-cycloalkanoyl, aryl, aralkyl, aroyl, hetaryl hetaralkyl and hetaroyl as defined above, optionally as a sole carbon source or as excess carbon source in the presence of normal nutrition buffers or bases such as nutrition agar gels, and using the enantiomeric excess of the formation of one of the diastereoisomers of the compound of formula I as a selection tool. Growth, incubation, and optionally activation or induction may be performed according to basic knowledge of a skilled person. If desired, the aldo-keto reductase may be isolated from the microorganisms in a manner known in the art. The method of identifying microorganisms containing an aldo-keto reductase being capable of reducing a ketolactone of formula II also is subject of the present invention.

(3)

Polypeptides having aldo-keto reductase activity useful in the process of the invention typically comprise a set of the following sequences

(i)

SX1X2X3GX4X5X6SX7X8IX9G (SEQ ID NO 1),
GX10X11X12X13DX14AX15X16Y (SEQ ID NO 2), and
LX17X18X19X20X21X22X23X24X25DX26X27X28X29H (SEQ ID NO 3),

or
(ii)

LX1X2X3GX4X5X6PX7X8GX9G (SEQ ID NO 4),
GX10X11X12X13DX14AX15X16Y (SEQ ID NO 2), and
LX17X18X19X20X21X22X23X24X25DX26X27X28X29H (SEQ ID NO 3),

wherein

X1 is K, P, S or G,
X2 is T, Q, S, R, L, or K,
X3 is A C, Q, S or T,
X4 is I, L, V or T,
X5 is R, H or K,
X6 is V, A, I or T,
X7 is P, C, V, or R,
X8 is L or I,
X9 is L, V, F or I,
X10 is G, V, I, H or S,
X11 is N, T or R,
X12 is C, F, E, L or A,
X13 is I, L, F or V,
X14 is T or V,
X15 is N, F, D or E,
X16 is S, V, F, I or A
X17 is R, Q, A, K, E or T,
X18 is K, R or H,
X19 is L or M
X20 is deleted or R
X21 is Q, R or D,

X22 is T, C, M or L,
X23 is D, S, D, P or E,
X24 is W, Y, K or H,
X25 is I, V, A or L,
X26 is I, L or V
X27 is L, F, Y or V,
X2 is Y, Q, I or F,
X29 is I, V, C, M or A,

wherein the sequences in each set (i) and (ii) are consecutively linked in the given order by an amino acid sequence having a length of at least 10 amino acids so as to form the catalytically active structure of an aldo-keto reductase.

Preferably, SEQ ID NO 2 in the above sets of sequences has to its C-terminal end further attached the amino acid sequence XcK (SEQ ID NO 2-XcK), wherein Xc is an amino acid sequence of 10 to 40, preferably 20 to 32, and more preferably 26 amino acid residues as defined below.

For the skilled person it is clear, that up to 5 single amino acid residues denoted as X amino acids (i.e. X1 to X29) can be deleted or added to each of the sequences

SEQ ID NO 1, SEQ ID NO 2, SEQ ID NO 3 and SEQ ID NO 4, to be still within the scope of the present invention. This applies to all sequences derived from sequences SEQ ID NO 1, SEQ ID NO 2, SEQ ID NO 3 and SEQ ID NO 4; as well as to respective changes of the corresponding polypeptide encoding nucleic acid sequences (for example SEQ ID NO 12 and SEQ ID NO 13) provided below.

(4) According to the invention, the polypeptide having aldo-keto reductase activity may comprise a consecutive order of the sequences SEQ ID NO 1, SEQ ID NO 2 and SEQ ID NO 3, or SEQ ID NO 4, SEQ ID NO 2 and SEQ ID NO 3, respectively, which are linked by or contain the amino acid sequences Xa, Xb, Xc, Xd and Xe, or the amino acid sequences Xa, Xb, Xc, Xd, Xf and Xg as defined below. In particular, the polypeptide having aldo-keto reductase activity may comprise the sequences:

**Xa**SX1X2X3GX4X5X6SX7X8IX9G**Xb**GX10X11X12X13DX14AX15X16

Y**Xc**K**Xd**LX17X18X19X20X21X22X23X24X25DX26X27X28X29H**Xe** (SEQ ID NO 5)

or

**Xa**LX1X2X3GX4X5X6PX7X8GX9G**Xb**GX10X11X12X13DX14AX15X16

Y**Xc**K**Xd**LX17X18X19X20X21X22X23X24X25DX26X27X28X29H**Xe** (SEQ ID NO 6)

wherein X1 to X29 are as defined above,
Xa is a pattern of 10 to 30 amino acid residues, preferably 20 amino acid residues,
Xb is a pattern of 20 to 40 amino acid residues, preferably 30 amino acid residues,
Xc is a pattern of 10 to 40 amino acid residues, preferably 26 amino acid residues,
Xd is a pattern of 10 to 45 amino acid residues, preferably 33 amino acid residues, and
Xe is a pattern of 221 to 271 amino acid residues, preferably 238 amino acid residues.

(5) Alternatively, the polypeptide having aldo-keto reductase activity may comprise one of the following sequences:

**Xa**SX1X2X3GX4X5X6SX7X8IX9G**Xb**GX10X11X12X13DX14AX15X16 Y**Xc**K**Xd**LX17X18X19X20X21X22X23X24X25DX26X27X28X29H**Xf**V**Xg**

(SEQ ID NO 7),

or

**Xa**LX1X2X3GX4X5X6PX7X8GX9G**Xb**GX10X11X12X13DX14AX15X16 Y**Xc**K**Xd**LX17X18X19X20X21X22X23X24X25DX26X27X28X29H**Xf**V**Xg**

(SEQ ID NO 8)

wherein X1 to X29 are as defined above,
Xa is a pattern of 10 to 30 amino acid residues, preferably 20 amino acid residues,
Xb is a pattern of 20 to 40 amino acid residues, preferably 30 amino acid residues, Xc is a pattern of 10 to 40 amino acid residues, preferably 26 amino acid residues,
Xd is a pattern of 10 to 45 amino acid residues, preferably 33 amino acid residues,
Xf is a pattern of 70 to 100 amino acid residues, preferably 85 amino acid residues, and Xg is a pattern of 140 to 170 amino acid residues, preferably 152 amino acid residues.

More preferably, Xc has the amino acid sequence

QNEESEIWIGEWMASRKLRDQIVIAT (SEQ ID NO 9).

Xa, thus, is an amino acid sequence attached to the N-terminus of SEQ ID NO:1 or SEQ ID NO:4 and preferably is the amino acid sequence between the methionine residue encoded by the start codon ATG of the sequence encoding the polypeptide having aldo-keto reductase activity and the N-terminus of SEQ ID NO:1 or SEQ ID NO:4, respectively.

Xb is an amino acid sequence linking SEQ ID NO:1 or SEQ ID NO:4, respectively, with SEQ ID NO:2.

Xc is an amino acid sequence linking SEQ ID NO:2 with the lysine (K) residue.

Xd is an amino acid sequence following the Lysine residue attached to the C-terminal end of Xc and linking said amino acid with the N-terminus of SEQ ID NO:3.

Xe is an amino acid sequence attached to the C-terminus of SEQ ID NO:3. This amino acid sequence optionally may comprise a protein-tag such as a polyhistidine-tag, for example a hexahistidine-tag, which is used for protein purification.

Xf is an amino acid sequence attached to the C-terminus of SEQ ID NO:3 and linking SEQ ID NO:3 with the valine (V) residue.

Xg is the amino acid sequence at the C-terminal end of the polypeptide having also-keto reductase activity following the valine residue attached to Xf. This amino acid sequence optionally may comprise a protein-tag such as a polyhistidine-tag, for example a hexahistidine-tag.

The polypeptide used in the process of the invention may be obtained using an expression vector having inserted a nucleotide sequence encoding said polypeptide having aldo-keto reductase activity. A proper host cell such as a microorganism is transformed with said expression vector containing the coding sequence for the polypeptide and the host cell is cultured under conditions where the polypeptide is expressed and the polypeptide produced by the host cell is recovered.

(6) For example, the nucleotide sequence encoding YNL331C comprising 1131 base pairs can be used in an expression vector:

atgactgacttgtttaaacctctacctgaaccacctaccgaattgggacgtctcagggttctt
tctaaaactgccggcataagggtttcaccgctaattctgggaggagcttcaatcggcgacgca
tggtcaggctttatgggctctatgaataaggaacaggcctttgaacttcttgatgctttttat
gaagctggaggtaattgtattgatactgcaaacagttaccaaaatgaagagtcagagatttgg
ataggtgaatggatggcatcaagaaaactgcgtgaccagattgtaattgccaccaagtttacc
ggagattataagaagtatgaagtaggtggtggtaaaagtgccaactactgtggtaatcacaag
cgtagtttacatgtgagtgtgagggattctctccgcaaattgcaaactgattggattgatata
ctttacattcactggtgggattatatgagttcaatcgaagaagttatggatagtttgcatatt
ttagttcagcagggcaaggtcctatatttaggagtatctgatacacctgcttgggttgtttct
gcggcaaattactacgctacatctcatggtaaaactccttttagcgtctatcaaggtaaatgg
aatgtattgaacagggactttgagcgtgatattattccaatggctaggcattttggtatggct
ctagccccatgggatgtcatgggaggtggaagatttcagagtaaaaaagcaatggaagaacgg
aagaagaatggagagggtctgcgtacttttgtgggtggccccgaacaaacagaattggaggtt
aaaatcagcgaagcattgactaaaattgctgaggaacatggaacagagtctgttactgctatc
gctattgcctatgttcgctctaaagcgaaaaatgttttcccattgattggaggaaggaaaatt
gaacatctcaagcagaacattgaggctttgagtattaaattaacaccggaacaaatagaatac
ctggaaagtattgttccttttgatgttggctttcccaaaagtttaataggagatgacccagcg
gtaaccaagaagctttcacccctcacatcgatgtctgccaggatagcttttgacaattag

(SEQ ID NO 12)

According to a further embodiment, the nucleotide sequence of a clone of YNL331C comprising 1170 base pairs can be used in an expression vector, wherein the native stop codon of the sequence encoding YNL331C is removed and the protein coding sequence is fused to a sequence encoding a C-terminal His-tag:

atgactgacttgtttaaacctctacctgaaccacctaccgaattgggacgtctcagggttctt
tctaaaactgccggcataagggtttcaccgctaattctgggaggagcttcaatcggcgacgca
tggtcaggctttatgggctctatgaataaggaacaggcctttgaacttcttgatgctttttat
gaagctggaggtaattgtattgatactgcaaacagttaccaaaatgaagagtcagagatttgg
ataggtgaatggatggcatcaagaaaactgcgtgaccagattgtaattgccaccaagtttacc
ggagattataagaagtatgaagtaggtggtaaaagtgccaactactgtggtaatcacaag
cgtagtttacatgtgagtgtgagggattctctccgcaaattgcaaactgattggattgatata
ctttacattcactggtgggattatatgagttcaatcgaagaagttatggatagtttgcatatt
ttagttcagcagggcaaggtcctatatttaggagtatctgatacacctgcttgggttgtttct
gcggcaaattactacgctacatctcatggtaaaactcctttagcgtctatcaaggtaaatgg
aatgtattgaacagggactttgagcgtgatattattccaatggctaggcattttggtatggct
ctagccccatgggatgtcatgggaggtggaagatttcagagtaaaaaagcaatggaagaacgg
aagaagaatggagagggtctgcgtacttttgtgggtggccccgaacaaacagaattggaggtt
aaaatcagcgaagcattgactaaaattgctgaggaacatggaacagagtctgttactgctatc
gctattgcctatgttcgctctaaagcgaaaaatgttttcccattgattggaggaaggaaaatt
gaacatctcaagcagaacattgaggctttgagtattaaattaacaccggaacaaatagaatac
ctggaaagtattgttccttttgatgttggctttcccaaaagtttaataggagatgacccagcg
gtaaccaagaagctttcacccctcacatcgatgtctgccaggatagcttttgacaatAAGCTT
GCGGCCGCACTCGAGCACCACCACCACCACCACTGA

(SEQ ID NO 13)

It is well known in the art that a single amino acid may be encoded by more than one nucleotide codon, and that the nucleotide sequence may be modified to produce an alternate nucleotide sequence that encodes the same peptide. Therefore, alternate embodiments of the present invention include alternate DNA sequences encoding peptides containing the amino acid sequences as previously described. DNA sequences encoding peptides containing the claimed amino acid sequence include DNA sequences which encode any combination of the claimed sequence and other amino acids located at the N-terminal or C-terminal end of the present amino acid sequences. It is to be understood that amino acid and nucleic acid sequences may include additional residues, particularly N- or C-terminal amino acids or 5' or 3' nucleotide sequences, and still be essentially as set forth in the sequences disclosed herein, as long as the sequence confers aldo-keto reductase activity upon the expressed polypeptide or protein.

Additional nucleic acid bases may be added either 5' or 3' to the nucleotide sequence encoding the polypeptide having aldo-keto reductase activity, and may be combined with other DNA sequences, such as promoters, polyadenylation signals, additional restriction enzyme sites, multiple cloning sites, other coding segments, and the like. Therefore, overall length of such a polynucleotide may vary considerably.

Expediently suitable polypeptides are presented below.

(7) More particularly, the polypeptide having aldo-keto reductase activity may comprise the following sequence:

TDLFKPLPEPPTELGRLRVLSKTAGIRVSPLILGGASIGDAWSGFMGSMNKEQAFELLDAFYE

AGGNCIDTANSYQNEESEIWIGEWMASRKLRDQIVIATKFTGDYKKYEVGGGKSANYCGNHKR

SLHVSVRDSLRKLQTDWIDILYIHWWDYMSSIEEVMDSLHILVQQGKVLYLG

(SEQ ID NO 10),

or a functionally equivalent variant thereof conferring aldo-keto reductase activity to the polypeptide used in the biotransformation.

(8) For example, the polypeptide having aldo-keto reductase activity used in the process of the invention may comprise or consist of the sequence:

TDLFKPLPEPPTELGRLRVLSKTAGIRVSPLILGGASIGDAWSGFMGSMNKEQAFELLDAFYE

AGGNCIDTANSYQNEESEIWIGEWMASRKLRDQIVIATKFTGDYKKYEVGGGKSANYCGNHKR

SLHVSVRDSLRKLQTDWIDILYIHWWDYMSSIEEVMDSLHILVQQGKVLYLGVSDTPAWVVSA

ANYYATSHGKTPFSVYQGKWNVLNRDFERDIIPMARHFGMALAPWDVMGGGRFQSKKAMEERK

KNGEGLRTFVGGPEQTELEVKISEALTKIAEEHGTESVTAIAIAYVRSKAKNVFPLIGGRKIE

HLKQNIEALSIKLTPEQIEYLESIVPFDVGFPKSLIGDDPAVTKKLSPLTSMSARIAFDNKLA

AALE (SEQ ID NO 11),

or a functionally equivalent variant thereof conferring aldo-keto reductase activity to the polypeptide used in the biotransformation, which comprises the known wild type sequence (wt) of YNL331C.

(9) Preferred aldo-keto reductases useful in the process of the invention are alcohol oxidoreductases belonging to class EC 1.1, in particular class EC 1.1.1, for example classes EC 1.1.1.1 and EC 1.1.1.2 and functionally equivalent variants thereof having the aldo-keto reductase activity of these enzymes.

(10) More preferred aldo-keto reductases are arylalcohol-dehydrogenases, in particular those from yeasts, for example from the genus Saccharomyces, or a functionally equivalent variant thereof.

(11) More preferably, the polypeptide having aldo-keto reductase activity is the arylalcohol-dehydrogenase YNL331C from Saccharomyces cerevisiae or a functionally equivalent variant thereof.

Further functionally equivalent variants of YNL331C suitable in the present process can be found by alignment to AKR9B1 (according to the nomenclature system of Jez and Penning, supra; systematic name YNL331C, standard name AAD14).

It is to be understood that a "functionally equivalent variant" of a polypeptide is not completely identical to the native protein. A variant of a polypeptide having aldo-keto reductase activity, for example, can be obtained by altering

the amino acid sequence by insertion, deletion or substitution of one or more amino acids. The amino acid sequence of the polypeptide or protein can be modified, for example, by substitution to create a polypeptide having substantially the same or improved qualities as compared to the native polypeptide. The substitution may be a conservative substitution. A "conservative substitution" is a substitution of an amino acid with another amino acid having a side chain that is similar in polar/nonpolar nature, charge, or size. The 20 essential amino acids can be grouped as those having nonpolar side chains (alanine, valine, leucine, isoleucine, proline, phenylalanine, and tryptophan), uncharged polar side chains (methionine, glycine, serine, threonine, cystine, tyrosine, asparagine and glutamine), acidic side chains (aspartate and glutamate), and basic side chains (lysine, arginine, and histidine). Conservative substitutions include, for example, N (Asp) to E, N or Q (Glu, Asn, or Gln); H (His) to K, R or F (Lys, Arg or Phe); N (Asn) to Q, N or E (Gln, Asp or Glu); and s (Ser) to c, Y or G (Cys, Thr or Gly).

To those of skill in the art, variant polypeptides can be obtained by substituting a first amino acid for a second amino acid at one or more positions in the polypeptide structure in order to affect biological activity. Amino acid substitutions may, for example, induce conformational changes in a polypeptide that result in increased biological activity.

A skilled person may also make substitutions, deletions or insertions in the amino acid sequence based on the hydrophilicity index or hydropathic index of the amino acids. A variant amino acid molecule of the present invention, therefore, has less than one hundred percent, but at least about fifty percent, and preferably at least about eighty to about ninety percent amino acid sequence homology or identity to the amino acid sequence of a polypeptide comprising the amino acid sequence of SEQ ID NO 7, SEQ ID NO 8, SEQ ID NO 10 or SEQ ID NO 11.

The term "amino acid residue" as far as it refers to variants of polypeptides having aldo-keto reductase activity also is intended to include D-form amino acids and modified amino acids. These substitutions are known to a skilled person, using known structural similarities between the molecules. The provided amino acid sequences are also intended to include any polypeptide sequence that may include additional amino acids residues, either N-terminal or C-terminal to the listed sequences, or both.

Typically, if the biotransformation is not carried out using whole microorganisms but using the enzyme as such, for example in the form of cell extracts, cell-free extracts or purified polypeptides (enzymes), the biotransformation is carried out in the presence of the cofactor required by the enzyme, typically NADH or NADPH, and, optionally, a cofactor regenerating system.

Expediently, the process for preparing the compound of formula I in a biotransformation by reacting a compound of formula II with an enzyme having aldo-keto reductase activity as mentioned above is carried out in the presence of a cofactor regenerating system such as the GDH system depicted in Scheme 1. Preferably, in the biotransformation the compound of formula Ia is obtained.

Scheme 1: Schematic reduction system with NAD(P)H regeneration, wherein R1 is as defined above)

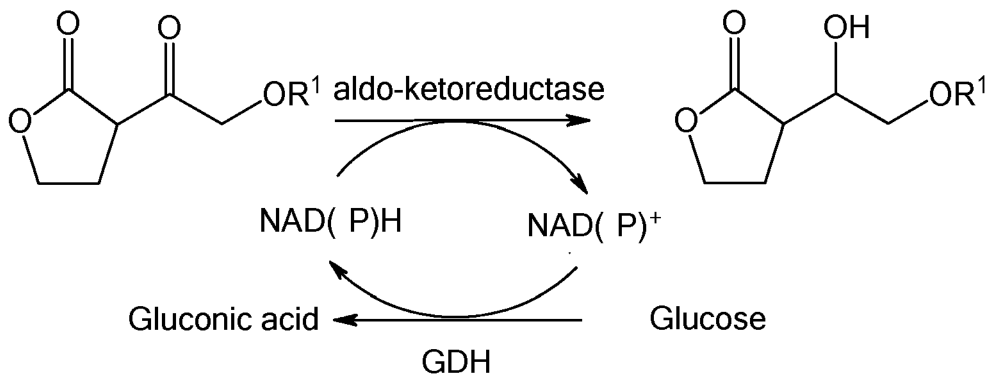


Thus, in a preferred embodiment the reduction of the compound of formula II to obtain the compound of formula I is carried out in the presence of nicotinamide adenine dinucleotide (NADH) or nicotinamide adenine dinucleotide phosphate (NADPH). Expediently, NAD(P)H is regenerated from NAD(P) and recycled in the process. Preferably the reaction is carried out in the presence of NAD(P)H and a cofactor regeneration system to regenerate NAD(P)H from NAD(P) as is commonly known by the skilled person. Several systems for regeneration of cofactors are known in the art for example, glucose and glucose dehydrogenase (GDH), formate dehydrogenase (FDH), chemically modified cofactor regeneration systems, light-driven cofactor regeneration systems, or immobilized forms of GDH and FDH as described by Wang et al., Biotechnol. Adv. 25, 2007, 369-384, which is incorporated herein by reference regarding cofactor regeneration.

The reactions when carried out in the presence of whole cells or cell lysates may not require the addition of cofactor and the regeneration system. In purified systems the addition of cofactor and regeneration system may be

necessary.

Also expediently, said cofactor regenerating system, for example for regenerating NAD(P)H from NAD(P), comprises an in-situ regenerating system selected from the group consisting of glucose dehydrogenase-glucose-gluconicacid (GDH) and formate-dehydrogenate (FDH) system.

Both preferred recycle systems, i.e. GDH and FDH may be used equivalently by the skilled person.

Specific improvements can be obtained by mutating the enzymes that catalyzes the desired reaction, or using a microorganism comprising a recombinant gene as the source of the aldo-keto reductase. The present invention includes such improvements of the enzyme regarding for example temperature stability, pH stability, enzymatic activity or reduced cofactor requirement.

Recombinant as mentioned above refers to a gene sequence that has been ligated in a vector (for example a plasmid) and has been introduced in a host cell system, which could be the parent strain or another suitable microorganism.

Optionally the enzymatic reduction is carried out in the range from pH 5 to 7, preferably in the range from pH 5.5 to 6.5, more preferred in the range from pH 5.8 to 6.2. Most preferred the reduction is carried out at pH 6.0.

Regarding the temperature, the biotransformation (enzymatic reduction) preferably is carried out at a temperature in the range from 20 to 45 °C, preferably in the range from 25 to 41 °C, more preferably in the range from 25 to 38 °C. Most preferred the biotransformation is carried out at about 31 °C.

The present invention also provides a method of identifying and selecting a microorganism or fungus expressing a polypeptide having aldo-keto reductase activity as defined above and suitable to perform the biotransformation in the process of the invention. Preferably the method for identifying comprising that the microorganism or fungus is contacted with the compound of formula II, wherein $R^1$ is selected from the group consisting of hydrogen, $C_{1-10}$-alkyl, $C_{2-11}$-acyl, $C_{3-8}$-cycloalkyl, $C_{4-9}$-cycloalkanoyl, aryl, aralkyl, aroyl, hetaryl hetaralkyl and hetaroyl, wherein the compound of formula II preferably is the sole carbon source, and wherein the enantiomeric excess of the formation of the desired diastereoisomer of the compound of formula I is used as a selection tool. Growing, incubation, optionally activation, can be performed to basic knowledge of a skilled person.

In a preferred embodiment of the invention, the product of the biotransformation is a compound of formula

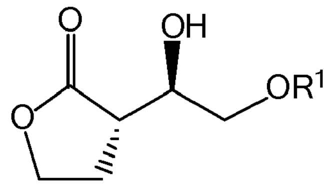

Ia,

wherein $R^1$ is selected from hydrogen, $C_{2-11}$-acyl, aryl, aralkyl, aroyl, hetaryl, hetaralkyl and hetaroyl.

(12) According to the invention, the compound of formula Ia, wherein $R^1$ is selected from hydrogen, $C_{2-11}$-acyl, $C_{4-9}$-cycloalkanoyl,aryl, aralkyl, aroyl, hetaryl, hetaralkyl and hetaroyl, may be further subjected to downstream chemistry to obtain a compound of the formula

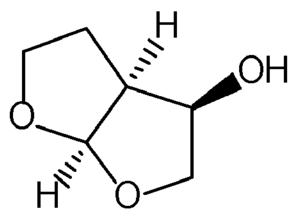

IIIa,

by cyclization. Said cyclization may comprise different routes depending on the residue $R^1$.

(13) According to a further embodiment of the invention, a compound of formula I, for example of formula I, preferably Ia, wherein $R^1$ is selected from the group consisting of $C_{2-11}$-acyl, $C_{4-9}$-cycloalkanoyl, aroyl and hetaroyl, is subjected to reduction by reaction with a complex metal hydride and subsequently is worked up in the presence of an acid, preferably a non-oxidizing acid, to obtain a compound of formula III, preferably IIIa.

Thus, a compound of formula Ia will be reduced to obtain a compound of formula IIIa, while a compound of formula Ib will be reduced to obtain a compound of formula IIIb. Expediently, said complex metal hydride is selected

from $NaBH_4$, $LiAlH_4$, or diisobutyllithium aluminum hydride (DIBAL).

Said reaction with a complex metal hydride expediently is carried out in a polar aprotic organic solvent. Preferably said organic solvent is selected from tetrahydrofuran (THF) and $CH_2Cl_2$.

To reduce isomerisation reactions, said reaction with a complex metal hydride is carried out at low temperatures, expediently at -40 °C or below, more preferably at about -70 °C or even lower.

Expediently, the acid is a non-oxidizing acid which is preferably selected from HCl, acetic acid, monochloro acetic acid, dichloro acetic acid, trichloro acetic acid, trifluoro acetic acid (TFA) and 3-chloro propionic acid. Preferably the acid is HCl or TFA.

In a most preferred embodiment, a compound of formula Ia, wherein $R^1$ is selected from the group consisting of $C_{2-11}$-acyl, $C_{4-9}$-cycloalkanoyl, aroyl and hetaroyl is reacted with a complex metal hydride, and subsequently is worked up in the presence of an acid, preferably a non-oxidizing acid, to obtain a compound of formula of IIIa.

(14) The present invention also provides for the preparation of a compound of formula

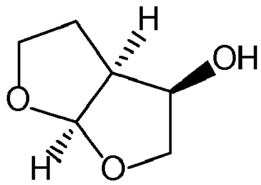

IIIa,

comprising the steps of

(i) reacting a compound of formula

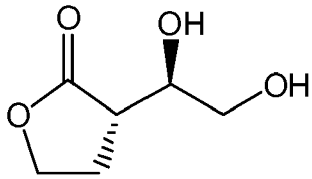

XVIa,

(i.e. compound Ia wherein $R^1$ is H) with a compound of formula

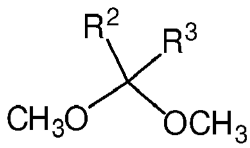

IV,

wherein $R^2$ and $R^3$ are independently selected from the group consisting of $C_{1-4}$-alkyl or $R^2$ and $R^3$ together form a $-(CH_2)_n-$ group wherein n is an integer from 3 to 5, to obtain a compound of formula

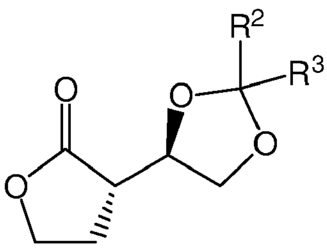

Va,

wherein $R^2$ and $R^3$ are as defined above,

(ii) reacting the compound of formula Va with a complex metal hydride to obtain a compound of formula

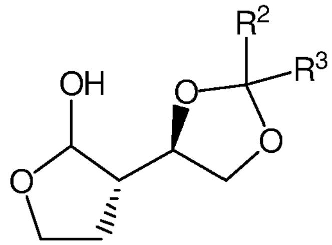

VIa,

wherein $R^2$ and $R^3$ are as defined above, and

(iii) hydrolyzing and cyclizing the compound of formula VIa in the presence of an acid preferably a non-oxidizing acid, to obtain the compound of formula IIIa.

(15) The compound of formula

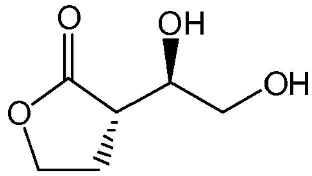

XVIa,

may either result directly from a biotransformation with an aldo-keto reductase of a compound of formula II, wherein $R^1$ is hydrogen, or it may be obtained by subjecting a compound of formula Ia, wherein $R^1$ is other than hydrogen and is selected from $C_{2-11}$-acyl, aryl, aralkyl, aroyl, hetaryl, hetaralkyl and hetaroyl, to a hydrogenation reaction to remove $R^1$.

Expediently, before cyclization the hydroxyl protecting group $R^1$ of the compound of formula I, which is other than hydrogen, can be removed by reacting with hydrogen in the presence of a catalyst. Preferably, said catalyst is a metal selected from the group consisting of Pd, Pt, Ir, Rh, and at least one of said metals is supported on a solid organic or inorganic carrier. Expediently said solid organic carrier is charcoal. Thus, a preferred catalyst is Pd/C, Pt/C, Ir/C, Rh/C, for example Pd/C with 5 or 10 wt-% Pd on charcoal. Suitable inorganic solid carriers are for example $TiO_2$, $SiO_4$, $NaHCO_3$, $Na_2CO_3$, $KHCO_3$, $K_2CO_3$ or zeolites.

Said conversion may be carried out without a conformational change.

Expediently, the compound of formula IV is selected from the group consisting of 2,2-dimethoxypropane, 2,2-dimethoxybutane, 2,2-dimethoxypentane, 3,3-dimethoxypentane, 2,2-dimethoxyhexane, 3,3-dimethoxyhexane, 3,3-dimethoxyheptane, 4,4-dimethoxyheptane, 3,3-dimethoxyoctane, 5,5-dimethoxynonane, 1,1-dimethoxycyclopentane, 1,1-dimethoxycyclohexane or 1,1-dimethoxycycloheptane

Expediently, the complex metal hydride in step (ii) above is selected from the group consisting of $NaBH_4$, $LiAlH_4$ and diisobutyllithium aluminum hydride (DIBAL).

Also expediently, the non-oxidizing acid in step (iii) above is selected from the group consisting of HCl, acetic acid, monochloro acetic acid, dichloro acetic acid, trichloro acetic acid, trifluoro acetic acid (TFA) and 3-chloro propionic acid. Preferably the acid is HCl or TFA.

(16) As mentioned above, provided is also a method of identifying a microorganism having aldo-keto reductase activity, said method comprising:

a) contacting a candidate microorganism with a compound of formula

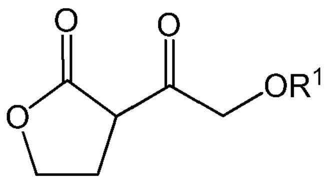

II,

wherein $R^1$ is selected from the group consisting of hydrogen, $C_{2-11}$-acyl, $C_{4-9}$-cycloalcanoyl, aryl, aralkyl, aroyl, hetaryl, hetaralkyl and hetaroyl, and

b) identifying a microorganism having aldo-keto reductase activity, wherein aldo-keto reductase activity is indicated by the formation of an enantiomeric excess of one of the diastereoisomers of the corresponding hydroxylactone.

Compounds of formula Ia or IIIa as they are obtained according to any of the processes referenced above, can be used for the preparation of a compound of formula

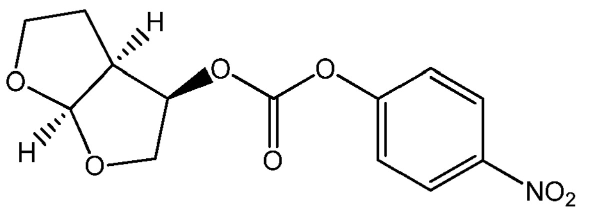

VII,

or a compound of formula

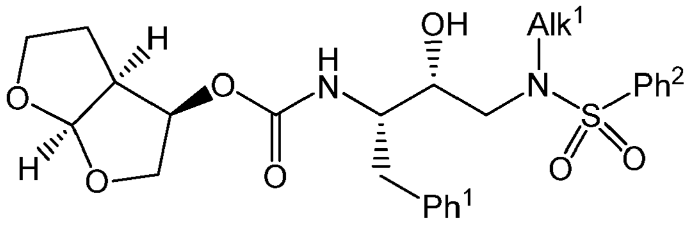

VIII,

derived therefrom, wherein $Alk^1$ is a linear or branched $C_{1-8}$-alkyl group, $Ph^1$ is phenyl and $Ph^2$ is 4-amino-phenyl, or $Ph^1$ is 4-[(2-methyl-1,3-thiazol-4-yl)methoxy]phenyl and $Ph^2$ is 1,3-benzodioxol-5-yl, or a physiologically acceptable salt thereof.

Preferably, $Alk^1$ is iso-butyl, $Ph^1$ is phenyl and $Ph^2$ is 4-amino-phenyl, or $Alk^1$ is *iso*-butyl, $Ph^1$ is 4-[(2-methyl-1,3-thiazol-4-yl)methoxy]phenyl and $Ph^2$ is 1,3-benzodioxol-5-yl, or a physiologically acceptable salt thereof.

The starting compound of formula II, wherein $R^1$ is selected from $C_{2-11}$-acyl, $C_{4-9}$-cycloalkanoyl, aryl, aralkyl, aroyl, hetaryl, hetaralkyl and hetaroyl, can be obtained for example by reacting a 2-halo acetate of formula

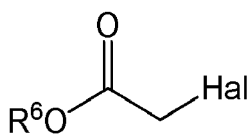

IX,

wherein $R^6$ is selected from $C_{2-11}$-acyl, $C_{4-9}$-cycloalkanoyl, aryl, aralkyl, aroyl, hetaryl, hetaralkyl and hetaroyl, and Hal is a halogen atom, with a compound of formula

$HOR^1$ X,

wherein $R^1$ is selected from $C_{2-11}$-acyl, $C_{4-9}$-cycloalkanoyl, aryl, aralkyl, aroyl, hetaryl, hetaralkyl and hetaroyl, to obtain a compound of formula

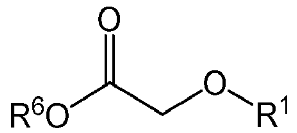

XI,

wherein $R^1$ and $R^6$ are as defined above, and said compound of formula XI is reacted with γ-butyrolactone.

Alternatively, the starting compound of formula II, wherein $R^1$ is selected from $C_{2-11}$-acyl, $C_{4-9}$-cycloalkanoyl, aroyl and hetaroyl, can be obtained by reacting a 2-halo acetate of formula

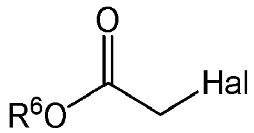

IX,

wherein $R^6$ is selected from $C_{2-11}$-acyl, $C_{4-9}$-cycloalkanoyl, aryl, aralkyl, aroyl, hetaryl, hetaralkyl and hetaroyl, and Hal is a halogen atom, with butyrolactone to obtain the compound of formula

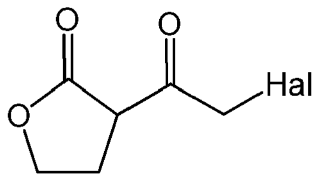

XII,

wherein Hal is as defined above, and said compound of formula XII is reacted with an acid of formula

$HOC(O)R^1$ XIII,

wherein $R^1$ is selected from $C_{2-11}$-acyl, $C_{4-9}$-cycloalkanoyl, aryl, aralkyl, aroyl, hetaryl, hetaralkyl and hetaroyl, or an acid anhydride of formula

$R^1C(O)\text{-}O\text{-}C(O)R^1$ XIV,

wherein $R^1$ is selected from $C_{2-11}$-acyl, $C_{4-9}$-cycloalkanoyl, aryl, aralkyl, aroyl, hetaryl, hetaralkyl and hetaroyl.

In another alternative, a compound of formula XI, wherein $R^1$ is selected from $C_{2-11}$-acyl, $C_{4-9}$-cycloalkanoyl, aroyl and hetaroyl and $R^6$ is as defined above, can be obtained by reacting a 2-hydroxy acetic acid ester of formula

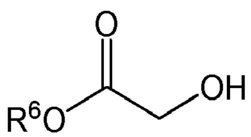

XV,

wherein $R^6$ is as defined above, with a suitable alkyl, cycloalkyl, aryl or hetaryl carbonyl halide or a carboxylic anhydride to obtain a compound of formula

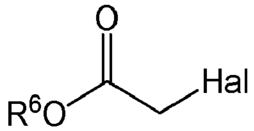

IX,

wherein $R^1$ is selected from $C_{2-11}$-acyl, $C_{4-9}$-cydoalkanoyl, aroyl and hetaroyl, and wherein Hal and $R^6$ are as defined above, and said compound of formula IX is reacted with γ-butyrolactone.

Expediently the reaction with γ-butyrolactone in each of the alternatives, i.e. the reaction with a compound of

formulae IX or XI is carried out in the presence of a strong non-nucleophilic base to avoid simple elimination instead of a Claisen condensation. Reacting a compound of formula IX with butyrolactone yields the compound of formula II, regardless whether $R^1$ is selected from $C_{2-11}$-acyl, $C_{4-9}$-cycloalkanoyl, aryl, aralkyl, aroyl, hetaryl, hetaralkyl or hetaroyl. A suitable non-nucleophilic base is lithium hexamethyldisilazide (LiHMDS, $[(CH_3)_3Si]_2NLi$). LiHMDS is commercially available but can also be freshly prepared for example by deprotonation of bis(trimethylsilyl)amine with butyl lithium. Other suitable bases other than LiHMDS are for example lithium diisopropylamide (LDA), NaHMDS, KHMDS, lithium tetramethylpiperidide (LiTMP), sodium tert-butoxide or potassium tert-butoxide.

In a preferred embodiment said Claisen condensation is carried out at a temperature below 0 °C. More preferably at a temperature of blow -50 °C. Examples were carried out at -60 or -70 °C.

Examples of suitable alkyl, cycloalkyl, aryl or hetaryl carbonyl halides are acetyl chloride, chloroacetyl chloride, butyryl chloride, isobutyryl chloride, phenylacetyl chloride, benzyl chloroformate (Cbz-Cl), propionyl chloride, benzoyl chloride, allyl chloroformate or tert-butyloxycarbonyl fluoride. Examples of suitable carboxylic anhydrides are *tert*-butoxycarbonyl anhydride, butyric anhydride, acetic anhydride or propionic anhydride.

The acylation (reaction with alkyl, cycloalkyl, aryl or hetaryl carbonyl halides or respective anhydrides) is expediently carried out in an aprotic solvent. Examples of suitable aprotic solvents are diisopropyl ether, pyridine, acetonitrile, acetone, $C_{1-6}$-alkyl acetates such as methyl acetate, ethyl acetate, or propyl acetate, dimethylformamide, diethylformamide, triethylamine, tetrahydrofuran, toluene, methylene chloride, N-methylpyrrolidone or mixtures thereof.

The acylation is expediently carried out at a temperature from -80 to 50 °C, preferably from 0 to 25 °C.

## Examples

The examples are not suited to demonstrate a limiting factor although not all claimed embodiments have been carried out experimentally due to the sheer mass of examples. Nevertheless, the examples constitute suitable means to demonstrate how all claimed embodiments can be carried out.

The examples are split up in three parts. Examples 1 to 7 are directed to the preparation of starting materials of the biotransformation (enzymatic reduction). Examples 8 to 16 are directed to the biotransformation. Examples 17 to 25 are directed to the downstream chemistry to obtain the compound of formula IIIa.

In examples and tables "*ee*" refers to enantiomeric excess, "*de*" refers to diastereoisomeric excess, whierein "*de*" refers to the the simple diastereoisometric ratio in percent of the syn and anti diastereoisomeres.

The enantiomeric excess is calculated by the following formulae:

$$ee\text{ syn} = (([R,R]-[S,S])/([R,R]+[S,S]) \text{ and } ee\text{ anti} = (([R,S]-[S,R])/([R,S]+[S,R])$$

Preparation of starting materials and reference compounds:

Example 1: Compound of formula XI, $R^1$ = benzyl, $R^6$ = ethyl

A reaction flask was charged with toluene (200 mL), followed by addition of NaH (70% purity, 105.4 g, 2.6 mol) and dropwise addition of benzyl alcohol (259 g, 2.4 mol, formula X, $R^1$ = benzyl)) at room temperature. The reaction mixture was heated at 30 °C while stirring for 1 h. The reaction mixture was cooled down to -40 °C and ethyl bromoacetate (400 g, 2.4 mol, formula IX, wherein $R^6$ = ethyl and Hal = Br) was added dropwise at a rate that the reaction temperature did not exceed -35 °C. After completion of the addition the reaction mixture was warmed to -30 °C over 2 h. Ice cold $H_2O$ (1.5 L) was added followed by addition of concentrated HCl until pH 6 was reached. The mixture was warmed to room temperature and the organic layer was collected. The aqueous layer was extracted with toluene (2×600 mL). The combined organic layers were evaporated. The residue was purified by vacuum distillation to afford 295.0 g of ethyl benzyloxyacetate (63% yield).

Example 2: Compound of formula II, $R^1$ = benzyl

In a reaction flask, lithium hexamethyldisilazide (LiHMDS, 1 M in toluene, 330 L, 0.33 mol) was charged followed by addition of anhydrous THF (400 mL). The solution was cooled down to -70 °C. γ-Butyrolactone (28.4 g, 0.33 mol) was added dropwise while the temperature of the reaction was kept at no more than 60 °C. Ethyl benzyloxyacetate (50.0 g, 0.26 mol, formula XI, $R^1$ = benzyl, $R^6$ = ethyl) was added dropwise. After the addition, the reaction mixture was stirred at -60 °C for 10 min. Ice-cold water (65 mL) was added, followed by addition of concentrated HCl to pH 3. The mixture was warmed to room temperature and the organic layer was collected. The aqueous layer was extracted with ethyl acetate (4×60 mL). The combined organic layers were evaporated and the residue was purified by flash column chromatography to afford 40.0 g (52% yield, based on γ-butyrolactone) of a compound of formula II, wherein $R^1$ is benzyl.

Example 3: Compound of formula II, $R^1$ = benzyl

The same reaction as in Example 2 was carried out using 1.28 equivalents of lithium hexamethyldisilazide (LiHMDS) to obtain a compound of formula II, wherein $R^1$ is benzyl, in 79% yield.

Example 4: Compound of formula XII, Hal = Cl

THF (120 mL) was added to a three-neck reaction flask under $N_2$ atmosphere and cooled down to -74 °C in an acetone/dry ice bath. LiHMDS (1 M solution in THF, 121 mL, 0.121 mol) was added to the solution in a fast stream. γ-Butyrolactone (8.5 mL, 0.11 mol) was added dropwise under temperature control (<-74 °C). The reaction mixture was stirred at -74 °C for 20 min. Ethyl chloroacetate (10.5 mL, 0.098 mol, formula IX, $R^6$ = ethyl, Hal = Cl) was added dropwise under temperature control (<-74 °C). The reaction mixture was stirred at -70 °C for 1 h. The reaction mixture was quenched by addition of 2 M HCl (150 mL) at -25 °C while keeping temperature control of the receiving reaction flask at <-20 °C. This mixture was stirred at -20 °C for 10 min and diluted with 50 mL $H_2O$ and poured into separating funnel and extracted with EtOAc (2×100 mL). The combined organic layers were washed with brine (2×60 mL), and evaporated to dark orange oil 16.26 g (approx 80% purity). The crude product, of formula XII (Hal = Cl), was used in the next reaction without further purification.

Example 5: Compound of formula II, $R^1$ = benzoyl

The crude chloroketone obtained in Example 4 (16.26 g, formula XII, wherein Hal = Cl) was placed in a 0.5 L reaction flask, dissolved with acetone (200 mL). Benzoic acid (18.11 g, 0.148 mol, formula XIII, $R^1$ = benzoyl) was added to the mixture. The mixture was stirred until all reagents had dissolved (approx. 10 min). Triethylamine (21.2 mL, 0.152 mol) was added producing a mildly exothermic reaction. A condenser was adapted to the reaction flask and the reaction mixture was stirred at 40 °C under $N_2$ atmosphere for 12 h. The reaction mixture was cooled down to room temperature and filtered through filter paper. The filter cake was washed with acetone (20 mL). The filtrate was evaporated to obtain an orange oil which was dissolved in ethyl acetate (100 mL). The ethyl acetate solution was washed with brine (2×30 mL), and evaporated to approx. half of the original volume (approx. 50 mL). To the light-brown solution, hexanes (20 mL) were added (turbidity was observed). The mixture was let stand at room temperature for 30 min and aged at 4 °C for 16 h. The suspension was filtered and the solids washed with hexanes (20 mL) at 4 °C. The solids were dried for 30 min to give 13.5 g of white solid (55.5% yield for two steps, examples 4 and 5).

Example 6: Compound of general formula I, $R^1$ = benzyl

The racemic alcohol of formula I ($R^1$ = benzyl) was obtained by subsequent reduction of the compound of formula II ($R^1$ = benzyl) with sodium borohydride. The four diastereoisomers where separated using a chiral HPLC column (Column: Chiralcel OD-H, 4.6 x 250 mm; Eluent: 90:10 *n*-heptane/ isopropyl alcohol; Flow: 1 mL/min, Detection: 215 nm, retention times: 16.29 min (syn diastereoisomer (*S*,*S*)), 18.96 min (syn diastereoisomer (*R*,*R*)), 21.52 min (desired anti diastereoisomer of formula Ia, (*S*,*R*)), and 27.02 min (anti diastereoisomer, (*R*,*S*)).

Example 7: Compound of general formula I, $R^1$ = benzoyl

The racemic alcohol of formula I, wherein $R^1$ is benzoyl was obtained by subsequent reduction of the compound of formula II ($R^1$ = benzoyl) with sodium borohydride. The four diastereoisomers where separated using a chiral HPLC column (Column: Chiralpak AD, 4.6 x 250 mm; Eluent: 65:35 n-heptane/isopropyl alcohol; Flow: 0.6 mL/min, Detection: 210 nm, Retention times: 11.46 min (anti diastereomer (*R*,*S*)), 12.18 min (syn diastereomer (*S*,*S*)), 13.16 min (anti diastereomer (*S*,*R*)), and 14.12 min (syn diastereomer (*R*,*R*)).

Enzymatic Examples:

General description for the enzymatic reduction (biotransformation, biocatalytic reduction) of a compound of formula II, wherein $R^1$ is defined in each example, using an aldo-keto reductase with GDH, glucose, gluconic acid cofactor regeneration system.

Example 8:

Aldo-keto reductase screening was carried out using benzyl ketolactone of formula II ($R^1$ = benzyl, 100 mg/mL), potassium phosphate buffer (100 mM, pH 7.0, containing NADH (2.5 mg/mL), NAD(P)H (2.5 mg/mL), also-keto reductase

(10 mg/mL). 96 well plates were incubated at 30 °C for 26 h at 900 rpm. The results of 16 different aldo-keto reductases (commercially available, for example, from Codexis Inc., 200 Penobscot Drive, Redwood City, CA 94063, USA) on benzyl ketolactone (formula II, $R^1$ = benzyl) are displayed in Table 1, wherein the Enzyme indicates the Catalog-No under which the enzyme is available or, in case of YNL331C, the enzyme name:

Table 1

| Enzyme | Conversion (%) | *ee* (syn) | *ee* (anti) | *de* (anti/syn) |
|---|---|---|---|---|
| KRED-125 | 58 | 49 (*S*,*S*) | 100 (*R*,*S*) | 80 (anti) |
| KRED-EXP-B1H-162 | 75 | 100 (*S*,*S*) | 100 (*S*,*R*) | 72 (anti) |
| KRED-EXP-B1I | 55 | 100 (*S*,*S*) | 100 (*S*,*R*) | 86 (anti) |
| KRED-EXP-B1J-163 | 71 | 100 (*S*,*S*) | 100 (*S*,*R*) | 80 (anti) |
| KRED-EXP-B1K-164 | 79 | 100 (*S*,*S*) | 100 (*S*,*R*) | 86 (anti) |
| KRED-EXP-B1M-166 | 50 | 100 (*S*,*S*) | 100 (*S*,*R*) | 86 (anti) |
| KRED-EXP-B1S-171 | 83 | n.a. | n.a. | n.a. |
| YSC2 | 88 | 16 (*R*,*R*) | 94 (*R*,*S*) | 87 (anti) |
| 42.10 | 76 | 37 (*R*,*R*) | 94 (*S*,*R*) | 91 (anti) |
| 51476 | 94 | 11 (*R*,*R*) | 91 (*R*,*S*) | 85 (anti) |
| KRED-NADH-106 | 50 | 74 (*S*,*S*) | 100 (*S*,*R*) | 85 (anti) |
| KRED-NADH-1112 | 64 | 64 (*R*,*R*) | 100 (*R*,*S*) | 68 (anti) |
| KRED-NADH-120 | 87 | 16(*S*,*S*) | 100 (*S*,*R*) | 65 (anti) |
| KRED-NADH124 | 70 | 100 (*S*,*S*) | 100 (*S*,*R*) | 70 (anti) |
| YNL3331 c | 46 | 31 (*S*,*S*) | 94 (*S*,*R*) | 83 (anti) |
| KRED-243 | 85 | 100 (*R*,*R*) | 68 (*R*,*S*) | 93 (anti) |

Values reported as percentage of area of the main peak; n.a. = data not available

For ee: The brackets (*R*,*R*) and (*S*,*S*) indicate the enantiomeric excess of the syn diastereomers, expressed in percent in terms of the major component (*R*,*R*) or (*S*,*S*) diastereomers, respectively, while the brackets (*R*,*S*) and (*S*, *R*) indicate the enantiomeric excess of the anti diastereomers, expressed in percent in terms of the major component (*R*,*S*) or (*S*,*R*) diastereomers, respecitively.

For de: The bracket (anti) indicates the ratio, expressed in percent, of anti diastereomers (*R*,*S* + *S*,*R*) over the syn diastereomers (*S*,*S* + *R*,*R*), while (syn) is the ratio, expressed in percent, of syn diastereomers (*R*,*R* + *S*,*S*) over the anti diastereomers (*R*,*S* + *S*,*R*).

Example 9:

Aldo-keto reductase screening has been carried out with benzoyl ketolactone according to example 9. The results of 5 different aldo-ketoreductases (Codexis Inc., 200 Penobscot Drive, Redwood City, CA 94063, USA) reacting with benzoyl ketolactone (formula II, $R^1$ = benzoyl) are displayed in Table 2.

Table 2

| Enzyme | conversion (%) | *ee* (syn) | *ee* (anti) | *de* (anti/syn) |
|---|---|---|---|---|
| KRED-125 | -- | 100 (*S*,*S*) | 100 (*S*,*R*) | 99 (syn) |
| KRED-EXP-A1N-144 | 60 | 91 *(R,R)* | 100 (*S*,*R*) | 100 (syn) |
| 42.10 | 67 | 90 *(R,R)* | 46 (*S*,*R*) | 97 (syn) |
| KRED-NADH-106 | 50 | 64 *(R,R)* | 75 (*R*,*S*) | 90 (syn) |
| YNL3331c | 55 | 67 *(R,R)* | 2 (*R*,*S*) | 81 (syn) |

Example 10: Biotransformation of a compound of formula II, $R^1$ is benzyl Biotransformation of benzyl ketolactone (formula II, $R^1$ = benzoyl) with 50% enzyme loading corresponding to 0.2 mg (0.5 mg/mL) of enzyme in 0.4 mL reaction volume. Results of obtained benzoyl hydroxylactone (formula I, $R^1$ = benzoyl) are displayed in Table 3.

Table 3: Conversion in percent at a substrat:enzyme ratio of 1:0.5

| Enzyme | Cofactor | 1 h | 18 h | 24 h |
|---|---|---|---|---|
| KRED-EXP-B1I | NADPH | 1% | 1% | 2% |
| KRED-EXP-B1J-163 | NADPH | 7% | 8% | 85% |
| KRED-EXP-B1K-164 | NADPH | 15% | 56% | 56% |
| KRED-EXP-B1M-166 | NADPH | 2% | 8% | 8% |
| YNL3331c | NADPH | 28% | 69% | 69% |
| 42.10 | NADH | 2% | 18% | 20% |
| KRED-NADH-106 | NADH | 54% | 93% | 93% |

Example 11: Biotransformation of a compound of formula II, $R^1$ is benzyl Biotransformation of benzoyl ketolactone (formula II, $R^1$ = benzyl) has been carried out according to example 9 but with 250% enzyme loading corresponding to 1 mg (2.5 mg/mL) of enzyme in 0.4 mL reaction volume. Results of obtained benzyl hydroxylactone (formula I, $R^1$ = benzyl) are displayed in Table 4.

Table 4: Conversion in percent at a substrate:enzyme ratio of 1:2.5

| Enzyme | Cofactor | 1 h | 18 h | 24 h |
|---|---|---|---|---|
| KRED-EXP-B1I | NADPH | 3% | 18% | 19% |
| KRED-EXP-B1J-163 | NADPH | 32% | 34% | 34% |
| KRED-EXP-B1K-164 | NADPH | 61% | 100% | 100% |
| KRED-EXP-B1M-166 | NADPH | 2% | 40% | 40% |
| YNL3331c | NADPH | 96% | 100% | 100% |
| 42.10 | NADH | 9% | 73% | 77% |
| KRED-NADH-106 | NADH | 92% | 96% | 96% |

Example 12: Biotransformation of a compound of formula II, $R^1$ is benzoyl Biotransformation of benzoyl ketolactone (0.4 mg per well, 0.4 g/L concentration, formula II, $R^1$ = benzoyl) with 50% enzyme loading corresponding to 0.2 mg (0.5 mg/mL) of enzyme in 0.4 mL reaction volume. Results of obtained benzoyl hydroxylactone (formula I, $R^1$ = benzoyl) are displayed in Table 5.

Table 5: Conversion in percent at a substrate:enzyme ratio of 1:0.5

| Enzyme | Cofactor | 2 h | 4.5 h | Extraction | *ee* | *de* |
|---|---|---|---|---|---|---|
| KRED-125 | NADPH | 100 | -- | 100 | 100 (*S*,*R*) | 98.9 (syn) |
| KRED-EXPA1N-144 | NADPH | 98 | 100 | 100 | 98.8 (*R*,*S*) | 98.2 (anti) |
| YNL3331c | NADPH | 71 | 78 | 79 | 87 (*R*,*S*) | 88.4 (anti) |
| KRED-NADH-106 | NADH | 20 | 40 | 63 | 90 (*R*,*S*) | 86 (anti) |

Example 13: Biotransformation of a compound of formula II, $R^1$ is benzoyl Biotransformation of benzoyl ketolactone (compound of formula II, $R^1$ is benzoyl) has been carried out according to example 12 but with 250% enzyme loading corresponding to 1 mg (2.5 mg/mL) of enzyme in 0.4 mL reaction volume. Results of obtained benzoyl hydroxylactone (compound of formula I, $R^1$ = benzoyl) are displayed in Table 6.

Table 6: Conversion in percent at a substrate:enzyme ratio of 1:2.5

| Enzyme | Enzyme | Cofactor | 2h | 4.5h | Extraction | *ee* | *de* |
|---|---|---|---|---|---|---|---|
| KRED-125 | KR-024 | NADPH | 100 | -- | 100 | 100 (*S*,*R*) | 98.9 (syn) |
| KRED-EXPA1N-144 | KR-044 | NADPH | 100 | -- | 100 | 98.8 (*R*,*S*) | 98.2 (anti) |
| YNL3331c | YNL3331 c | NADPH | 98 | --- | 99 | 87 (*R*,*S*) | 88.4 (anti) |
| KRED-NADH-106 | KR-102 | NADH | 85 | 94 | 94 | 90 (*R*,*S*) | 86 (anti) |

Example 14: Biotransformation of a compound of formula II, $R^1$ is benzyl Biotransformation was carried out with enzyme YNL3331C (380 mg) and substrate benzyl ketolactone (compound of formula II, $R^1$ is benzyl, 760 mg, dissolved in 7.5 mL DMSO) in potassium buffer pH 6.0 in 150 mL reaction volume and incubated at 36 °C, 270 rpm for 2 h to reach 100% conversion. After extraction with MTBE (2×150 mL) 96% crude yield was obtained with 97.6% ee and 89.1% *de*, 17.9:1 *dr.*

Example 15: Biotransformation of a compound of formula II, $R^1$ is benzoyl

In a reaction flask equipped with pH-stat and thermocouple, 10 mM phosphate buffer pH 6.5 was charged (92 mL) and warmed to 34 °C. An aldo-keto reductase preparation of YNL3331C according to example 14 (16 mL), glucose (7.28 g), glucose dehydrogenase (GDH, 30 mg) and NAD(P)H (50 mg) were added to the buffer solution. Benzoyl ketolactone (compound of formula II, $R^1$ = benzoyl, 10 g) was added as solution in DMSO (5 mL) resulting in a milky reaction mixture. The reaction mixture was stirred at 120 rpm, 35 °C for 14 h (typical conversion >98%, monitored by HPLC). To the reaction mixture ethyl acetate (30 mL) was added and stirred at 35 °C for 10 min. The organic layer was collected. The aqueous layer was extracted with ethyl acetate (3×40 mL). The combined organic layers were stirred with celite® (1 g) for 2 min and filtered through a celite® bed. The solids were washed with ethyl acetate (10 mL). The filtrate was evaporated to approx. one-third of the original volume (40 mL) and the solution cooled down to 0 °C. Hexanes (10 mL) were added to the concentrated ethyl acetate solution (precipitation was observed). The suspension was filtered through filter paper and the solids washed with hexanes (5 mL). The white solid was dried for 30 min to give 7.06 g of benzoyl hydroxy lactone (70% crude yield, compound of formula I, $R^1$ = benzoyl).

Example 16: Biotransformation of benzoyl ketolactone of formula II, $R^1$ = benzoyl

In a reaction flask equipped with pH-stat and thermocouple, phosphate buffer (92 mL, 10 mM, pH 6.0) was charged and warmed to 34 °C. The enzyme preparation as described in example 14 (4 mL), Glucose (1.46 g, 8.1 mmol), glucose dehydrogenase (GDH, 17 mg) and NADPH (12 mg, 0.01 mmol) were added to the buffer solution. Benzoyl ketolactone (compound of formula II, $R^1$ = benzoyl, 1 g, 4.03 mmol) was added as solution in DMSO (4 mL) resulting in a milky reaction mixture. The reaction mixture was stirred at 120 rpm, 35 °C for 14 h (typical conversion >98%, monitored by HPLC). Ethyl acetate (30 mL) was added to the reaction mixture and stirred at 35 °C for 10 min. The mixture was allowed to separate into two phases and the organic phase was collected. The aqueous layer was extracted with ethyl acetate (2×15 mL). The combined organic phases were stirred with celite® (1 g) for 2 min and filtered through a celite® bed. The solids were washed with ethyl acetate (5 mL). The filtrate was evaporated to approx. one-fourth of the original volume (approx. 15 mL) and the solution cooled down to 0 °C. Hexanes (5 mL) were added to the concentrated ethyl acetate solution to precipitate the product. The suspension was filtered through filter paper and the solids washed with hexanes (5 mL). The white solid was dried for 30 min to give 830 mg of product (compound of formula I, $R^1$ = benzoyl, 82% crude yield).

Downstream chemistry (A) to (C) starting from compounds of general formula I obtained by the present biotransformation.

(A) Direct cyclization:

The route using direct cyclization of a compound of formula I in the presence of a complex metal hydride is particularly advantageous and only possible in case of using a starting compound of formula I, wherein $R^1$ is selected from $C_{2-11}$-acyl, $C_{4-9}$-cycloalcanoyl, aroyl and hetaroyl, preferably wherein $R^1$ is benzoyl.

Example 17: 6H-furofuranol (preparation of compound of formula IIIa)

Reduction of the hydroxy lactone of formula Ia, wherein is $R^1$ = benzoyl, with DIBAL-H (4 eq.) in $CH_2Cl_2$ at -78

°C, wherein the crude reaction was treated with drops of TFA afforded 6H-furofuranol of formula IIIa in 23% yield.

Example 18: 6H-furofuranol (preparation of compound of formula IIIa)

Reduction of the hydroxy lactone of formula Ia, wherein is $R^1$ = benzoyl, with DIBAL-H (3.3 eq.) in $CH_2Cl_2$ at -78 °C followed by acidic work-up with TFA afforded 6H-furofuranol of formula IIIa in 23-25% yield.

Example 19: 6H-furofuranol (preparation of compound of formula IIIa)

To a stirred solution of benzoyl hydroxyactone of formula Ia ($R^1$ = benzoyl, 401.9 mg, 1.61 mmol) in THF (20 mL) at -74 °C under nitrogen atmosphere, DIBAL-H (1 M soln. in $CH_2Cl_2$, 6.5 mL, 6.5 mmol) was slowly added under temperature control (<-70 °C). The reaction mixture was stirred at -74 °C for 1 h. The reaction mixture was quenched by addition of MeOH (0.5 mL) and stirred at -65 °C for 10 min. The quenched reaction mixture was treated with HCl (6 N, 5 mL) and stirred for 10 min at about 5 °C. This mixture was extracted with MTBE (3×25 mL). The combined organic layers were concentrated to approx. one-fourth of the original volume (approx. 7 mL) and treated with hexanes (5 mL). The solids were collected by filtration and washed with hexanes (82 mL) to give 104.7 mg (50% yield) of the 6H-furofuranol of formula IIIa.

(B) Acetonide route:

Preferably, the acetonide route is used for compounds of formula I wherein $R^1$ is aralkyl or hetaralkyl, preferably benzyl or heteroarylmethyl, respectively.

Example 20: Compound of formula Va ($R^2$ = $R^3$ = methyl)

To a solution of the hydroxylactone of formula Ia ($R^1$ = benzyl, 685 mg, 2.9 mmol) in acetone (15 mL), 2,2-dimethoxypropane (3.6 mL, formula IV, $R^2$ = $R^3$ = methyl) and Amberlyst 15® (dry type, 20 mg) were added at room temperature. Then, Pd/C (10% Pd on charcoal by weight, 120 mg) was added in one portion. The reaction was stirred at room temperature under hydrogen (1 bar) for 8 h. The suspension was filtered through a celite® pad and the solids were washed with acetone (15 mL). The filtrate was evaporated and placed in a high vacuum pump for 1 h. The residue was purified by flash column chromatography on a silica gel column. Elution with n-heptane/ethyl acetate (2:1, v:v) afforded a waxy solid (385 mg, 71 %) of acetonide lactone of formula Va ($R^2$ = $R^3$ = methyl). In samples during the reaction the compound of formula XVIa (i.e. compound of formula Ia with $R^1$ = hydrogen) could be identified as intermediate between removal of $R^1$ and before ketal formation with the compound of formula IV to obtain the compound of formula Va.

Example 21: Compound of formula VIa ($R^2$ = $R^3$ = methyl)

In a reaction flask equipped with a thermocouple and under a stream of $N_2$, acetonide lactone of formula Va ($R^2$ = $R^3$ = methyl) (370 mg, 1.99 mmol) was dissolved in toluene (3.6 mL). The flask was cooled down to -74 °C (dry ice/acetone). DIBAL-H (1 M in toluene, 2.19 mL, 1.1 eq.) was added dropwise over 15 min through a syringe while the temperature of the reaction was maintained at below -70 °C. The reaction mixture was stirred at -74 °C for 90 min. The reaction was quenched by addition of ethyl acetate (2 mL) followed by $NH_4Cl$ saturated solution (3 mL). The reaction mixture slowly warmed to 10 °C. Anhydrous $MgSO_4$ (3 g), ethyl acetate (20 mL) and celite® (3 g) were added. This mixture was filtered through a celite® pad and the solids washed with ethyl acetate (60 mL). The filtrate was evaporated to give a residue (356 mg, 95% crude yield) of the acetonide lactol of formula VIa ($R^2$ = $R^3$ = methyl).

Example 22: 6H-furofuranol (compound of formula IIIa)

The lactol of formula VIa ($R^2$ = $R^3$ = methyl) (343 mg, 1.82 mmol) was dissolved in THF (8 mL) in a reaction flask. HCl (6N, 5 drops) was added slowly over the solution under vigorous stirring at room temperature. The reaction mixture was stirred at room temperature for 23 hours. Then the reaction mixture was treated with $K_2CO_3$ (3.5 g) and further stirred for 20 min. The suspension was filtered through filter paper and the solids washed with THF (2×10 mL). The filtrate was evaporated until dryness. The residue was purified by flash column chromatography on a silica gel column. Elution with ethyl acetate/heptane (2:1, v:v), and evaporation of the fractions, gave afforded 6H-furofuranol of formula IIIa in 61% yield (145 mg).

Example 23:

According to example 21, the compound of formula Ia, wherein $R^1$ is benzyl was reacted with DIBAL-H to obtain the compound of formula IIa.

(C) Double hydration route:

Alternatively, the compound of formula I, wherein $R^1$ is an aralkyl or hetaralkyl group, can be cyclized by a first hydration of the hydroxy lacton ring with a complex hydride as defined above to obtain the corresponding hydroxy lactol and a second hydration with hydrogen in the presence of a metal and an acid to remove the aralkyl or hetaralkyl group and to cyclize the product in the presence of an acid as mentioned above to obtain the respective compound of formula III. Preferably, said catalyst is a metal selected from the group consisting of Pd, Pt, Ir, Rh, and at least one of said metals is supported on a solid organic or inorganic carrier. Expediently said solid organic carrier is charcoal. Thus, a preferred catalyst is Pd/C, Pt/C, Ir/C, Rh/C, for example Pd/C with 5 or 10 wt-% Pd on charcoal. Suitable inorganic solid carriers are for example $TiO_2$, $SiO_4$, $NaHCO_3$, $Na_2CO_3$, $KHCO_3$, $K_2CO_3$ or zeolites. As mentioned above, preferably the acid is a non-oxidizing acid.

The product of the first hydration of a compound of formula I (hydroxy lacton), wherein $R^1$ is aralkyl or hetaralkyl, is a hydroxy lactol, i.e. a compound of formula

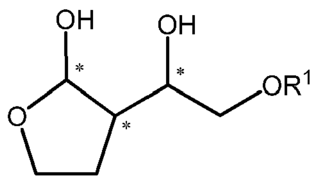

XVII,

wherein $R^1$ is aralkyl or hetaralkyl, in a preferred embodiment the product of the first hydration is a compound of formula

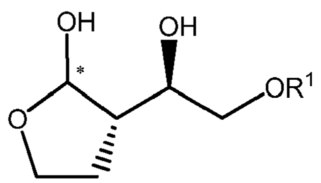

XVIIa,

wherein $R^1$ is aralkyl or hetaralkyl.

Example 24:

To a stirred solution of benzyl hydroxylactone of formula Ia ($R^1$ = benzyl, 7.06 g, 29.88 mmol) in $CH_2Cl_2$ (80 mL) at -74 °C under nitrogen atmosphere, diisobutyllithium aluminum hydride (DIBAL-H, 65 wt-% soln. in toluene, 13.67 mL, 44.82 mmol, 1.5 equivalents) was slowly added under temperature control (<70°C). The reaction mixture was stirred at -74 °C for 2 h. The reaction mixture was quenched by addition of MeOH (2 mL) and stirred at -65 °C for 10 min. The quenched reaction mixture was treated with saturated $NH_4Cl$ (10 mL) and stirred for 30 min without control temperature (typically 5 °C). The mixture was filtered through celite®. The solids were washed with $CH_2Cl_2$ (20 mL). The organic layer was separated and dried with anhydrous $Na_2SO_4$ (2 g) and filtered through cotton plug. The cotton plug was washed with $CH_2Cl_2$ (5 mL).

Example 25: 6H-furofuranol (compound of formula IIIa)

The filtrate of example 23 comprising the compound of formula XVIIa ($R^1$ = benzyl) was transferred to a hydrogenation vessel. Palladium on carbon (Pd/C, 10 wt-%, 100 mg) was added and formic acid (0.5 g) was added. The vessel was connected into the hydrogenator. The mixture was purged with $N_2$ and then purged once with $H_2$ and loaded with $H_2$ at 2 bar. The reaction mixture was stirred at room temperature at 2 bar $H_2$ pressure for 10 h. The crude

was filtered through celite® and the solids washed with ethyl acetate (10 mL). The solvent was evaporated to give a colorless oil which was purified by flash column chromatography to give 2.33 g of a colorless compound of formula IIIa (60% for two steps, i.e. examples 24 and 25).

## Claims

**1.** A process for the preparation of a compound of formula

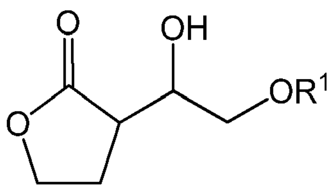

I,

wherein $R^1$ is selected from the group consisting of hydrogen, $C_{2-11}$-acyl, $C_{4-9}$-cycloalkanoyl, aryl, aralkyl, aroyl, hetaryl, hetaralkyl and hetaroyl, said process comprising a biotransformation of a compound of formula

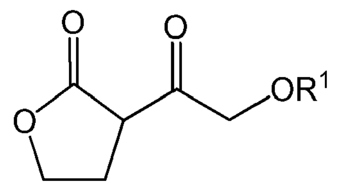

II,

wherein $R^1$ is as defined above, wherein said biotransformation is carried out using a polypeptide having aldo-keto reductase activity or a microorganism containing same.

**2.** The process of claim 1 for the preparation of the diastereoisomer of formula

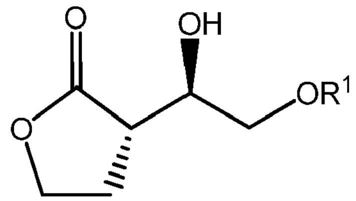

Ia,

wherein $R^1$ is as defined above.

**3.** The process of claims 1 or 2, wherein the polypeptide having aldo-keto reductase activity comprises a set of the following sequences

(i)

SX1X2X3GX4X5X6SX7X8IX9G (SEQ ID NO 1),
GX10X11X12X13DX14AX15X16Y (SEQ ID NO 2), and
LX17X18X19X20X21X22X23X24X25DX26X27X28X29H (SEQ ID NO 3),

or
(ii)

LX1X2X3GX4X5X6PX7X8GX9G (SEQ ID NO 4),
GX10X11X12X13DX14AX15X16Y (SEQ ID NO 2), and
LX17X18X19X20X21X22X23X24X25DX26X27X28X29H (SEQ ID NO 3),

wherein

X1 is K, P, S, or G,
X2 is T, Q, S, R, L, or K,
X3 is A, C, Q, S or T,
X4 is I, L, V or T,
X5 is R, H or K,
X6 is V, A, I or T,
X7 is P, C, V, or R,
X8 is L or I,
X9 is L, V, F or I,
X10 is G, V, I, H or S,
X11 is N, T or R,
X12 is C, F, E, L or A,
X13 is I, L, F or V,
X14 is T or V,
X15 is N, F, D or E,
X16 is S, V, F, I or A
X17 is R, Q, A, K, E or T,

X18 is K, R or H,
X19 is L or M
X20 is deleted or R
X21 is Q, R or D,
X22 is T, C, M or L,
X23 is D, S, D, P or E,
X24 is W, Y, K or H,
X25 is I, V, A or L,
X26 is I, L or V
X27 is L, F, Y or V,
X28 is Y, Q, I or F,
X29 is I, V, C, M or A, and wherein the sequences in each set (i) and (ii) are consecutively linked in the given order by an amino acid sequence having a length of at least 10 amino acids.

**4.** The process of any one of claims 1 to 3, wherein the polypeptide having aldo-keto reductase activity comprises the following sequence:

XaSX1X2X3GX4X5X6SX7X8IX9GXbGX10X11X12X13DX14AX15X16

YXcKXdLX17X18X19X20X21X22X23X24X25DX26X27X28X29HXe

(SEQ ID NO 5)

or

XaLX1X2X3GX4X5X6PX7X8GX9GXbGX10X11X12X13DX14AX15X16

YXcKXdLX17X18X19X20X21X22X23X24X25DX26X27X28X29HXe

(SEQ ID NO 6)

wherein x1 to x29 are as defined above,
xa is a pattern of 10 to 30 amino acid residues, preferably 20 amino acid residues,
xb is a pattern of 20 to 40 amino acid residues, preferably 30 amino acid residues,
xc is a pattern of 10 to 40 amino acid residues, preferably 26 amino acid residues,
xd is a pattern of 10 to 45 amino acid residues, preferably 33 amino acid residues, and
xe is a pattern of 221 to 271 amino acid residues, preferably 238 amino acid residues.
xf is a pattern of 70 to 100 amino acid residues, preferably 85 amino acid residues, and xg is a pattern of 140 to 170 amino acid residues, preferably 152 amino acid residues.

**5.** The process of any one of claims 1 to 3, wherein the polypeptide having aldo-keto reductase activity comprises the following sequence:

XaSX1X2X3GX4X5X6SX7X8IX9GXbGX10X11X12X13DX14AX15X16

YXcKXdLX17X18X19X20X21X22X23X24X25DX26X27X28X29HXfVXg

(SEQ ID NO 7),

or

XaLX1X2X3GX4X5X6PX7X8GX9GXbGX10X11X12X13DX14AX15X16

YXcKXdLX17X18X19X20X21X22X23X24X25DX26X27X28X29HXfVXg

(SEQ ID NO 8)

wherein x1 to x29 are as defined above,
xa is a pattern of 10 to 30 amino acid residues, preferably 20 amino acid residues,
xb is a pattern of 20 to 40 amino acid residues, preferably 30 amino acid residues,
xc is a pattern of 10 to 40 amino acid residues, preferably 26 amino acid residues,
xd is a pattern of 10 to 45 amino acid residues, preferably 33 amino acid residues,

**6.** The process of any one of claims 1 to 5, wherein the polypeptide having aldo-keto reductase activity comprises an amino acid sequence encoded by a DNA sequence selected from the group consisting of:

a) a DNA having the following sequence

atgactgacttgtttaaacctctacctgaaccacctaccgaattgggacgtctcaggg

ttctttctaaaactgccggcataagggtttcaccgctaattctgggaggagcttcaat

cggcgacgcatggtcaggctttatgggctctatgaataaggaacaggcctttgaactt

cttgatgctttttatgaagctggaggtaattgtattgatactgcaaacagttaccaaa

atgaagagtcagagatttggataggtgaatggatggcatcaagaaaactgcgtgacca

gattgtaattgccaccaagtttaccggagattataagaagtatgaagtaggtggtggt

aaaagtgccaactactgtggtaatcacaagcgtagtttacatgtgagtgtgagggatt

ctctccgcaaattgcaaactgattggattgatatactttacattcactggtgggatta

tatgagttcaatcgaagaagttatggatagtttgcatattttagttcagcagggcaag

gtcctatatttaggagtatctgatacacctgcttgggttgtttctgcggcaaattact
acgctacatctcatggtaaaactccttttagcgtctatcaaggtaaatggaatgtatt
gaacaggactttgagcgtgatattattccaatggctaggcattttggtatggctcta
gccccatgggatgtcatgggaggtggaagatttcagagtaaaaaagcaatggaagaac
ggaagaagaatggagagggtctgcgtacttttgtgggtggccccgaacaaacagaatt
ggaggttaaaatcagcgaagcattgactaaaattgctgaggaacatggaacagagtct
gttactgctatcgctattgcctatgttcgctctaaagcgaaaaatgttttcccattga
ttggaggaaggaaaattgaacatctcaagcagaacattgaggctttgagtattaaatt
aacaccggaacaaatagaatacctggaaagtattgttccttttgatgttggctttccc
aaaagtttaataggagatgacccagcggtaaccaagaagctttcacccctcacatcga
tgtctgccaggatagcttttgacaattag

(SEQ ID NO 12)

or a strand complementary to this sequence;
b) DNA sequences which hybridize under stringent conditions to the DNA sequence defined in a) or a strand complementary thereto and being capable of expressing a polypeptide having aldo-keto reductase activity;
or a fragment or fragments of said amino acid sequences conferring aldo-keto reductase activity to said polypeptide.

**7.** The process of any one of claims 1 to 6, wherein the polypeptide having aldo-keto reductase activity comprises the following sequence:

TDLFKPLPEPPTELGRLRVLSKTAGIRVSPLILGGASIGDAWSGFMGSMNKEQAFELL
DAFYEAGGNCIDTANSYQNEESEIWIGEWMASRKLRDQIVIATKFTGDYKKYEVGGGK
SANYCGNHKRSLHVSVRDSLRKLQTDWIDILYIHWWDYMSSIEEVMDSLHILVQQGKV
LYLG

(SEQ ID NO 10),

or a functionally equivalent variant thereof conferring aldo-keto reductase activity to the polypeptide used in the biotransformation.

**8.** The process of any one of claims 1 to 7, wherein the polypeptide having aldo-keto reductase activity comprises or consists of the following sequence:

TDLFKPLPEPPTELGRLRVLSKTAGIRVSPLILGGASIGDAWSGFMGSMNKEQAFELL

DAFYEAGGNCIDTANSYQNEESEIWIGEWMASRKLRDQIVIATKFTGDYKKYEVGGGK

SANYCGNHKRSLHVSVRDSLRKLQTDWIDILYIHWWDYMSSIEEVMDSLHILVQQGKV

LYLGVSDTPAWVVSAANYYATSHGKTPFSVYQGKWNVLNRDFERDIIPMARHFGMALA

PWDVMGGGRFQSKKAMEERKKNGEGLRTFVGGPEQTELEVKISEALTKIAEEHGTESV

TAIAIAYVRSKAKNVFPLIGGRKIEHLKQNIEALSIKLTPEQIEYLESIVPFDVGFPK

SLIGDDPAVTKKLSPLTSMSARIAFDNKLAAALE

(SEQ ID NO 11),

or a functionally equivalent variant of this sequence having aldo-keto reductase activity.

**9.** The process of any one of claims 1 to 8, wherein the polypeptide having aldo-keto reductase activity is an alcohol oxidoreductase belonging to class EC 1.1 or a functionally equivalent variant thereof.

**10.** The process of claim 9, wherein the polypeptide having aldo-keto reductase activity is an arylalcohol-dehydrogenase, in particular an arylalcohol-dehydrogenase derived from yeast, preferably from the genus Saccharomyces, or a functionally equivalent variant thereof.

**11.** The process of claim 9 or 10, wherein the polypeptide having aldo-keto reductase activity is arylalcohol-dehydrogenase YNL331C from Saccharomyces cerevisiae or a functionally equivalent variant thereof.

**12.** The process of any one of claims 1 to 11, further comprising subjecting a compound of formula

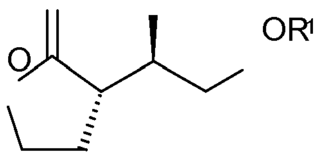

Ia,

wherein $R^1$ is selected from the group consisting of hydrogen, $C_{2-11}$-acyl, $C_{4-9}$-cycloalkanoyl, aryl, aralkyl, aroyl, hetaryl, hetaralkyl and hetaroyl, to a cyclization reaction to obtain a compound of formula IIIa

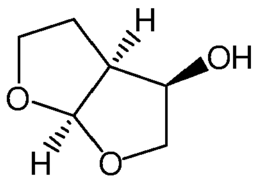

IIIa.

**13.** The process of any one of claims 1 to 11, further comprising subjecting a compound of formula

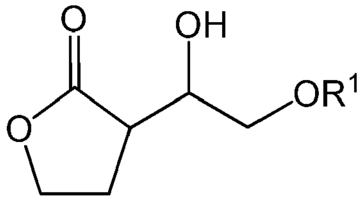

I,

wherein $R^1$ is selected from the group consisting of $C_{2-11}$-acyl, $C_{4-9}$-cycoalcanoyl, aroyl and hetaroyl, to reduction using a complex metal hydride and to subsequent treatment with an acid to obtain a compound of formula

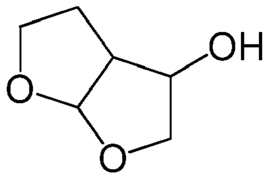

III.

**14.** The process of any one of claims 1 to 11, further comprising the steps of

(i) reacting a compound of formula XVIa (compound Ia wherein $R^1$ is hydrogen)

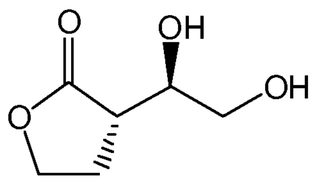

XVIa,

with a compound of formula

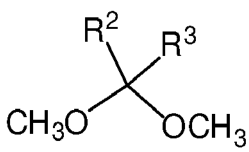

IV,

wherein $R^2$ and $R^3$ are independently selected from the group consisting of $C_{1-4}$-alkyl or $R^2$ and $R^3$ together form a $-(CH_2)_n-$ group wherein n is an integer from 3 to 5,
to obtain a compound of formula

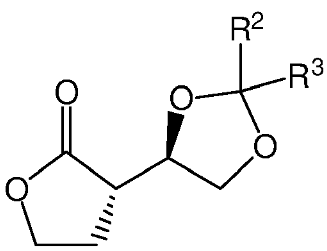

Va,

wherein $R^2$ and $R^3$ are as defined above,

(ii) reacting the compound of formula Va with a complex metal hydride to obtain a compound of formula

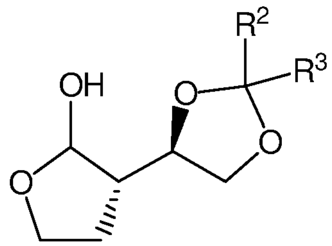

VIa,

wherein $R^2$ and $R^3$ are as defined above, and
(iii) hydrolyzing and cyclizing the compound of formula VIa in the presence of an acid to obtain the compound of formula

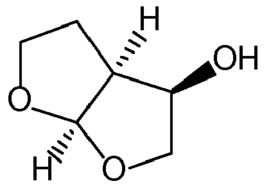

IIIa.

**15.** The process of claim 14, wherein the compound of formula XVIa is obtained by subjecting a compound of of formula

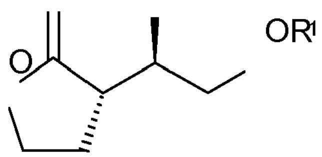

Ia,

wherein $R^1$ is selected from the group consisting of $C_{2-11}$-acyl, $C_{4-9}$-cycloalkanoyl, aryl, aralkyl, aroyl, hetaryl, hetaralkyl and hetaroyl, to a hydrogenation reaction.

**16.** A method of identifying a microorganism having aldo-keto reductase activity, said method comprising:

a) contacting a candidate microorganism with a compound of formula

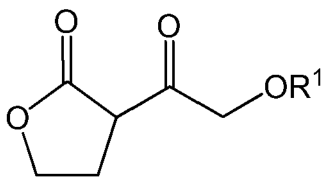

II,

wherein $R^1$ is selected from the group consisting of hydrogen, $C_{2-11}$-acyl, $C_{4-9}$-cycloalcanoyl, aryl, aralkyl, aroyl, hetaryl, hetaralkyl and hetaroyl, and
b) identifying a microorganism having aldo-keto reductase activity, wherein aldo-keto reductase activity is indicated by the formation of an enantiomeric excess of one of the diastereoisomers of the corresponding hydroxylactone.

Europäisches Patentamt
European Patent Office
Office européen des brevets

# EUROPEAN SEARCH REPORT

Application Number
EP 12 15 7752

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y<br>A | US 2004/162340 A1 (IKEMOTO TETSUYA [JP] ET AL) 19 August 2004 (2004-08-19)<br>* Reaction of cpd. (XXI) to cpd. (XXII); example 22 * | 16<br>1-15 | INV.<br>C07D307/33<br>C07D493/04<br>C12P17/04 |
| Y | ZHANG J ET AL: "Efficient NADPH recycling in enantioselective bioreduction of a ketone with permeabilized cells of a microorganism containing a ketoreductase and a glucose 6-phosphate dehydrogenase", ADVANCED SYNTHESIS & CATALYSIS, WILEY VCH VERLAG, WEINHEIM, DE, vol. 348, no. 4, 1 January 2006 (2006-01-01), pages 429-433, XP002416331, ISSN: 1615-4150, DOI: 10.1002/ADSC.200505439<br>* the whole document * | 16 | |
| A | YAN NI ET AL: "Biocatalytic properties of a recombinant aldo-keto reductase with broad substrate spectrum and excellent stereoselectivity", APPLIED MICROBIOLOGY AND BIOTECHNOLOGY, SPRINGER, BERLIN, DE, vol. 89, no. 4, 28 October 2010 (2010-10-28), pages 1111-1118, XP019880527, ISSN: 1432-0614, DOI: 10.1007/S00253-010-2941-4<br>* the whole document * | 16 | TECHNICAL FIELDS SEARCHED (IPC)<br>C07D<br>C12P |
| A | EP 2 348 120 A1 (UNIV WIEN [AT]; UNIV GRAZ TECH [AT]) 27 July 2011 (2011-07-27)<br>* the whole document * | 1-16 | |

The present search report has been drawn up for all claims

1

| Place of search | Date of completion of the search | Examiner |
|---|---|---|
| The Hague | 1 August 2012 | Fritz, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 15 7752

This annex lists the patent family members relating to the patent documents cited in the above-mentioned European search report.
The members are as contained in the European Patent Office EDP file on
The European Patent Office is in no way liable for these particulars which are merely given for the purpose of information.

01-08-2012

| Patent document cited in search report | Publication date | Patent family member(s) | Publication date |
|---|---|---|---|
| US 2004162340 A1 | 19-08-2004 | AU 2003275675 A1 | 29-07-2004 |
| | | EP 1589018 A1 | 26-10-2005 |
| | | US 2004162340 A1 | 19-08-2004 |
| | | US 2005256322 A1 | 17-11-2005 |
| | | WO 2004060895 A1 | 22-07-2004 |
| EP 2348120 A1 | 27-07-2011 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005063209 A
- WO 2007126812 A
- US 20040127727 A
- EP 1532127 A
- EP 1589018 A


### Non-patent literature cited in the description


- **JEZ, J.M. ; PENNING, T.M.** *Chem. Biol. Interact.,* 2001, vol. 130-132, 499-525
- **JEZ, J.M. ; FLYNN, T.G. ; PENNING, T.M.** *Biochem. Pharmacol.,* 1997, vol. 54, 639-647
- **WANG et al.** *Biotechnol. Adv.,* 2007, vol. 25, 369-384